# EUROPEAN PATENT SPECIFICATION

(11) **EP 2 437 786 B1**
(45) Date of publication and mention of the grant of the patent: **18.05.2016**
(21) Application number: 10726604.1
(22) Date of filing: 01.06.2010
(51) Int. Cl.: A61K 47/48

(54) **PRODRUGS CONTAINING ALBUMIN BINDING PROBE**
PRODRUGS MIT ALBUMINBINDENDER SONDE
PROMÉDICAMENTS CONTENANT UNE SONDE SE LIANT À L'ALBUMINE

(30) Priority: 01.06.2009 US 182910 P
(43) Date of publication of application: 11.04.2012
(73) Proprietor: Yeda Research and Development Co. Ltd., 76100 Rehovot (IL)
(72) Inventor: SHECHTER, Yoram, 76100 Rehovot (IL); FRIDKIN , Matityahu, 76284 Rehovot (IL)
(74) Representative: Vossius & Partner Patentanwälte Rechtsanwälte mbB
(86) International application number: PCT/IL2010/000433
(87) International publication number: WO 2010/140148

(56) References cited:
- WO-A2-2004/089280
- WO-A2-2005/117984
- WO-A2-2006/105201
- WO-A2-2011/013128
- SASSON K ET AL: "Engineering prolonged-acting prodrugs employing an albumin-binding probe that undergoes slow hydrolysis at physiological conditions" JOURNAL OF CONTROLLED RELEASE, ELSEVIER, AMSTERDAM, NL LNKD- DOI:10.1016/J.JCONREL.2009.10.028, vol. 142, no. 2, 3 March 2010 (2010-03-03) , pages 214-220, XP026905283 ISSN: 0168-3659 [retrieved on 2009-10-30]
- SHECHTER YORAM ET AL: "Delivery of Neuropeptides from the Periphery to the Brain: Studies with Enkephalin", ACS CHEMICAL NEUROSCIENCE, AMERICAN CHEMICAL SOCIETY, US, vol. 1, no. 5, 19 May 2010 (2010-05-19), pages 399-406, XP008130979, ISSN: 1948-7193, DOI: 10.1021/CN100001J [retrieved on 2010-02-16]

## Description

### TECHNICAL FIELD

The present invention relates to albumin-binding probes capable of converting short-lived amino-containing drugs into inactive reactivable prodrugs having prolonged lifetime profiles *in vivo.*

### BACKGROUND ART

Most polypeptide drugs, in particular nonglycosylated proteins of molecular mass less than 50 kDa, are short-lived species *in vivo* having circulatory half lives of 5-20 min. The short lifetime of proteins *in vivo* is attributed to several mechanisms including glomerular filtration in the kidneys and proteolysis at several levels (Goodman and Gilman, 1995).

An attractive strategy for improving clinical properties of small protein drugs has come to be known as PEGylation (or pegylation). Pegylated proteins are long-lived species *in vivo* having higher stability and aqueous solubility, as well as lower immunogenicity and antigenicity, compared with the corresponding non-pegylated proteins, and the potential for specific cell targeting (Clark *et al.,* 1996; Delgado *et al.,* 1992; Reddy, 2000; Bailon *et al.,* 2001). In spite of those profound clinical properties obtained by pegylation, only a limited number of pegylated proteins is in clinical use. The covalent attachment of polyethylene glycol (PEG) chains to proteins often results in a marked reduction in biological/pharmacological potency, and this is obviously valid with regard to low molecular-weight drugs (Marcus *et al.,* 2008). This deficiency can principally be overcome by introducing the PEG chain via a chemical bond that is sensitive to hydrolysis, or can be cleaved enzymatically by serum proteases or esterases. Clearly, a consistent rate of hydrolysis is crucial, and a prerequisite condition is therefore that the hydrolysis of the PEG chains from the conjugate is to take place at a slow rate and in a homogenous fashion *in vivo.*

In recent years we have developed a strategy of reversible pegylation according to which PEG chains containing sulfhydryl-moieties are covalently linked to amino groups of drug compounds through the heterobifunctional agent (2-(3-(2,5-dioxo-2,5-dihydro-1H-pyrrol-1-yl)propanamido)-9H-fluoren-9-yl)methyl 2,5-dioxopyrrolidin-1-yl carbonate, herein designated MAL-Fmoc-OSu, or 7-(3-(2,5-dioxo-2,5-dihydro-1H-pyrrol-1-yl)propanamido)-9-(((2,5-dioxopyrrolidin-1-yloxy)carbonyloxy)methyl)-9H-fluorene-2-sulfonic acid, herein designated MAL-FMS-OSu, which contains a spontaneously hydrolyzable bond. The inactive conjugate thus obtained is transformed into a long-acting prodrug that gradually releases its pharmacologically active constituent upon incubation at physiological conditions (Marcus *et al.,* 2008; Nesher *et al.,* 2008; Peleg-Shulman *et al.,* 2004; Shechter *et al.,* 2005a; Tsubery *et al.,* 2004).

In particular, US 7,585,837 discloses a compound of the formula (X)ₙ-Y, where Y is a moiety of a drug bearing at least one functional, e.g., amino, group, and X is a radical such as: 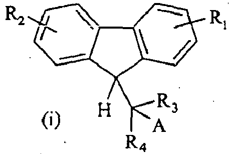 wherein R₁ is a radical containing a protein or a polymer carrier moiety; R₂ is selected from hydrogen, (C₁-C₈)alkyl, (C₁-C₈)alkoxy, (C₁-C₈)alkoxyalkyl, (C₆-C₁₀)aryl, (C₁-C₈)alkaryl, (C₆-C₁₀)ar(C₁-C₈)alkyl, halogen, nitro, -SO₃H, -SO₂NHR, amino, ammonium, carboxyl, PO₃H₂, or OPO₃H₂, and R is selected from hydrogen, (C₁-C₈)alkyl or (C₆-C₁₀)aryl; R₃ and R₄, the same or different, are each selected from hydrogen, (C₁-C₈)alkyl or (C₆-C₁₀)aryl; A is a covalent bond when the radical is linked to a carboxyl, phosphate or mercapto group of the drug Y, or A is OCO-when the radical is linked to an amino or hydroxyl group of the drug Y; n is an integer of at least one, and pharmaceutically acceptable salts thereof. As disclosed in this publication, said protein carrier may be, e.g., albumin such as human serum albumin (HSA), said polymer carrier may be, e.g., a linear or branched PEG, and said drug containing at least one free amino group may be a non-peptidic drug or a peptide or protein drug, most preferably of low or medium molecular weight.

Since albumin is long-lived *in vivo,* drugs and endogenous substances that tightly associate with albumin have lower clearance rates than that of the unbound substances, and exhibit prolonged lifetime profiles *in vivo* (Taylor and Granger, 1984). Long-chain fatty acids (LCFAs) bind tightly to albumin (Carter and Ho, 1994), and this provided the impetus for designing an insulin derivative, in which LCFA-like probe has been integrated into the insulin molecule (Kurtzhals *et al.,* 1995, 1996, 1997). The optimal derivative thus obtained (insulin-detemir) possessing protracted action *in vivo,* in part due to its associating affinity to endogenous albumin (Kurtzhals *et al.,* 1995, 1996, 1997).

Insulin detemir (Levemir®, NovoNordisk) is a long-acting human insulin analog with up to 24 hours duration of action. In particular, it is an insulin analog in which the amino acid threonine in position B30 is omitted and myristic acid has been attached to the amino acid lysine in position B29 via the acyl group, i.e., N^{*∈*B29}-tetradecanoyl des(B30) insulin. In the blood, insulin-detemir binds to albumin through the alkyl residue of the myristic acid and it is then slowly dissociated from this complex.

Insulin detemir as well as other similar derivatives of insulin are disclosed in US Patent Nos. 5,750,497, 6,011,007 and 6,869,930, and in US Patent Publication Nos. 20040110664 and 20060030518. These publications disclose an insulin derivative in which (i) the amino acids at positions A21 and B3 are, independently, any amino acid residue which can be coded for by the genetic code except Lys, Arg and Cys; (ii) the amino acid at position B1 is Phe or is deleted; (iii) the amino acid at position B30 is any amino acid residue which can be coded for by the genetic code except Lys, Arg and Cys, or is deleted; and (iv) the amino *∈*-amino group of Lys^{B29} is substituted with an acyl group having at least 10 carbon atoms or a lipophilic substituent having at least 6 carbon atoms, wherein the insulin derivative is a Zn²⁺ complex and the Zn²⁺ complex of the insulin derivative is more water soluble than the insulin derivative without Zn²⁺. The technology disclosed in these publications is directed to insulin derivatives only, wherein the lipophilic substituent is linked to the insulin derivative via an amino group on the insulin molecule, preferably the ∈-amino, of the amino acid lysine at position B29, and the insulin derivative is bound to albumin, upon administration, mainly via binding groups present in the albumin molecule capable of binding aliphatic chains.

US 7,186,797 discloses polypeptide conjugates having extended half life *in vivo,* comprising a polypeptide conjugated to a binding moiety having affinity for albumin. The binding moiety disclosed has two arms, wherein each one of these arms binds to albumin via a certain linking group that is either an aryl moiety or a non-aromatic moiety having 1-10 carbon atoms.

WO 2008053360 discloses portable albumin binders, capable of binding to albumin through a functional group that is negatively charged or may be deprotonated to yield a negative charge, e.g., a carboxyl group, which are said to be useful for improving the pharmacokinetic properties of diagnostic or therapeutic agents, e.g., by increasing their circulation lifetime. {page 4a}

WO 2005/117984 relates to an albumin-binding compound consisting essentially of the following elements: a spacer group, a water-soluble bridging group, a fatty acid chain and an acidic group characterized in that the acidic group is attached to the distal end of the fatty acid chain. WO 2005/117984 also provides an albumin-binding compound to which one or more biologically active moieties may be attached.

WO 2004/089280 concerns reversible pegylated drugs which may be prepared by derivatizing free functional groups of the drugs selected from amino, hydroxyl, mercapto, phosphate and/or carboxyl with groups which are sensitive to mild basic conditions such as 9-fluorenylmethoxycarbonyl (Fmoc) or 2-sulfo-9-fluorenylmethoxycarbonyl (FMS), to which group a PEG moiety is attached. In these pegylated drugs, the PEG moiety and the drug residue are not linked directly to each other, but rather both residues are linked to different positions of the scaffold Fmoc or FMS structure that is highly sensitive to bases and is removable under physiological conditions.

### SUMMARY OF INVENTION

Contrary to the concept disclosed in the aforesaid US 7,585,837, according to which a polymer carrier, e.g., PEG, or a protein carrier, e.g., albumin, is covalently linked to a drug via a heterobifunctional agent such as FMS or Fmoc, the present invention is based on a concept according to which a long chain fatty acid (LCFA) like albumin-binding compound is covalently linked to a short-lived amino-containing drug to form a drug conjugate capable of non-covalent association with albumin *in vivo,* i.e., a long-lived prodrug that gradually releases the pharmacologically active constituent.

In particular, it has been found, in accordance with the present invention, that by introducing a certain LCFA like albumin-binding compound, in particular, the 16-(1-(3-(9-(((2,5-dioxopyrrolidin-1-yloxy) carbonyloxy)-methyl)-7-sulfo-9H-fluoren-2-ylamino)-3-oxopropyl)-2,5-dioxopyrrolidin-3-ylthio) hexadecanoic acid, herein designated SuO-FMS-MAL-S-(CH₂)₁₅-COOH, to various short-lived amino-containing drugs such as insulin, exendin-4, gentamicin, Factor VIIa and Factor VIII, a conjugate having associating affinity to albumin is formed. The albumin-associating affinity of the conjugate formed is in the range of Ka=2.0-2.6x10⁵ M⁻¹, i.e., sufficient to turn short-lived molecules to long-lived species *in vivo.* Furthermore, although low molecular-weight drugs suffer from a massive loss of pharmacological potency upon conjugation, such drugs, when conjugated to said albumin-binding compound, regain their full potency upon incubation at physiological conditions.

In one aspect, the present invention thus relates to a compound of the formula I: 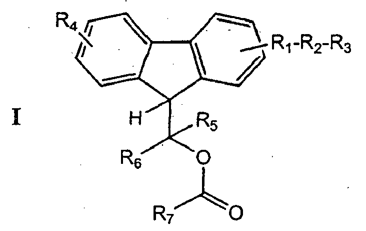 wherein
R₁ is selected from -NH-, -NH-CO-, -NH-CO-NH-, -S-, -SO₂NH- -O-,-OCO-, -CO-NH-, -CS-NH-, -CO(CH₂)₁₋₄-, or -R₈-CO-, wherein R₈ is (C₁-C₈)alkyl optionally interrupted by a heteroatom selected from O, S or N;
R₂ is selected from 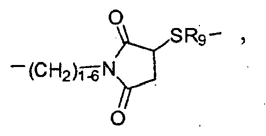 or R₉;
R₃ is an acidic group having at least one hydroxyl group such as-COOH, -SO₃H or -O-PO₃H₂;
R₄ is an electron withdrawing group such as -SO₃H, -CN, -CO-(C₁-C₈)alkyl, -CO-(C₆-C₁₀)aryl, -NO₂, -OPO₃H₂, -N(R)₃⁺, -SO₂NH₂, or halogen, wherein R is selected from (C₁-C₈)alkyl or (C₆-C₁₀)ar(C₁-C₈)alkyl;
R₅ and R₆, each independently is selected from hydrogen, -(C₁-C₈)alkyl or-(C₆-C₁₀)aryl;
R₇ is a leaving group such as -O-(CH₂)₂-CN, -Cl, 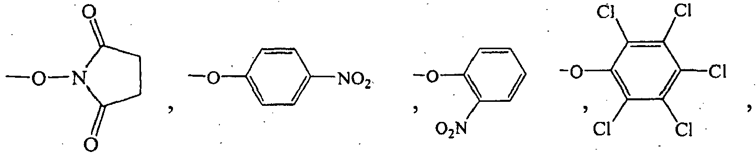 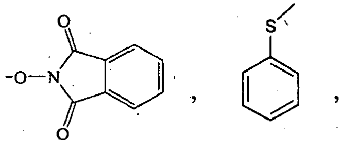 and 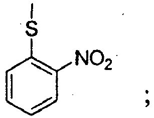 and R₉ is selected from (C₁₃-C₂₀)alkylene, (C₁₃-C₂₀)alkenylene or (C₁₃-C₂₀)alkynylene, optionally interrupted by (i) a heteroatom selected from S, O, or N, or more than one identical or different heteroatoms selected from S or N, and/or (ii) at least one group selected from -NH-CO-, -CO-NH-, -N(C₁-C₈alkyl)-, -N(C₆-C₁₀aryl)-, or -(C₆-C₁₀)arylene-diyl-, wherein said alkenylene or alkynylene comprises one or more double or triple bond, respectively, and said one or more double or triple bond is not a terminal double or triple bond.

In another aspect, the present invention relates to a conjugate of the formula II: 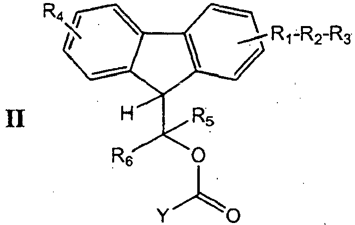 wherein
Y is a moiety of a drug containing at least one amino group, linked through said at least one amino group;
R₁ is selected from -NH-, -NH-CO-, -NH-CO-NH-, -S-, -SO₂NH- -O-,-OCO-, -CO-NH-, -CS-NH-, -CO(CH₂)₁₋₄-, or -R₈-CO-, wherein R₈ is (C₁-C₈)alkyl optionally interrupted by a heteroatom selected from O, S or N;
R₂ is selected from 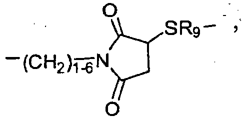 or R₉;
R₃ is an acidic group having at least one hydroxyl group such as - COOH, -SO₃H or -O-PO₃H₂;
R₄ is an electron withdrawing group such as -SO₃H, -CN, -CO-(C₁-C₈)alkyl, -CO-(C₆-C₁₀)aryl, -NO₂, -OPO₃H₂, -N(R)₃⁺, -SO₂NH₂, or halogen, wherein R is selected from (C₁-C₈)alkyl or (C₆-C₁₀)ar(C₁-C₈)alkyl;
R₅ and R₆, each independently is selected from hydrogen, -(C₁-C₈)alkyl or - (C₆-C₁₀)aryl; and
R₉ is selected from (C₁₃-C₂₀)alkylene, (C₁₃-C₂₀)alkenylene or (C₁₃-C₂₀)alkynylene, optionally interrupted by (i) a heteroatom selected from S, O, or N, or more than one identical or different heteroatoms selected from S or N, and/or (ii) at least one group selected from -NH-CO-, -CO-NH-, -N(C₁-C₈alkyl)-, -N(C₆-C₁₀aryl)- or -(C₆-C₁₀)arylene-diyl-, wherein said alkenylene or alkynylene comprises one or more double or triple bond, respectively, and said one or more double or triple bond is not a terminal double or triple bond. Further embodiments of the compound of formula I or the conjugate of formula II are defined in the claims and set out below.

Thus, in a further aspect, the present invention provides a pharmaceutical composition comprising a conjugate of the formula II as defined above, i.e., a conjugate obtained by nucleophilic substitution of a compound of formula I with an amino group of the drug Y, or a pharmaceutically acceptable salt thereof, and a pharmaceutically acceptable carrier. The pharmaceutical compositions of the present invention can be used for treatment of various diseases, disorders and conditions, in which administration of the drug Y might be useful.

Thus, in still a further aspect, the present invention provides a conjugate of formula II, as defined above, wherein the drug Y is insulin, obtained by nucleophilic substitution of a compound of formula I, as defined above, with an amino group of insulin for use in the treatment of diabetes mellitus or hyperglycemia.

In yet a further aspect, the present invention provides a conjugate of formula II, as defined above, wherein the drug Y is exendin-4, obtained by nucleophilic substitution of a compound of formula I, as defined above, with an amino group of exendin-4 for use in the treatment of insulin-dependent diabetes mellitus, non-insulin-dependent diabetes mellitus, or gestational diabetes mellitus, or for prevention of hyperglycemia.

In still another aspect, the present invention provides a conjugate of formula II, as defined above, wherein the drug Y is gentamicin, obtained by nucleophilic substitution of a compound of formula I, as defined above, with an amino group of gentamicin for use in the treatment of a bacterial infection, preferably a bacterial infection caused by Gram-negative bacteria.

In yet another aspect, the present invention provides a conjugate of formula II, as defined above, wherein the drug Y is Factor VIIa or Factor VIII, obtained by nucleophilic substitution of a compound of formula I, as defined above, with an amino group of Factor VIIa or Factor VIII, respectively for use in Factor VIIa or Factor VIII therapy, respectively.

### BRIEF DESCRIPTION OF DRAWINGS

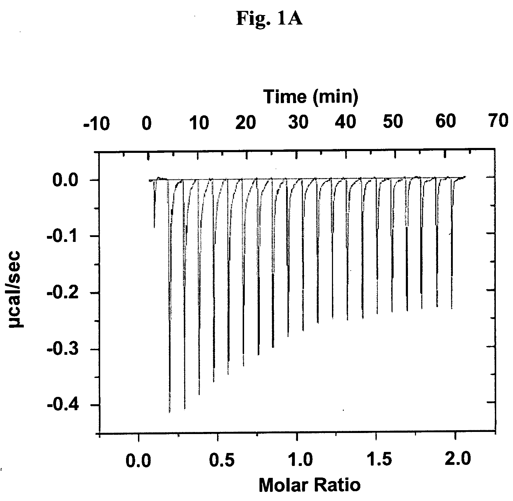

**Figs. 1A-1B** show the binding affinity of insulin-detemir to human serum albumin (HSA) as determined by ITC-200. The processed data has been derived from 20 automatic injections (2.4 µl each, **1A**) of insulin-detemir (400 µM in phosphate buffer saline, PBS, buffer, pH 7.4) into the sample cell containing HSA at a concentration of 10 *µ*M in PBS buffer, and was translated to a binding isotherm **(1B)**. Data: HSA_NDH; model: OneSites; chi^2/DoF=2.235x10⁴; N=0.693±0.0430 sites; K=6.87x10⁴±1.60x10⁴ M⁻¹; ΔH=-4575±406.7 cal/mol; ΔS=6.79 (cal/mol)/deg.

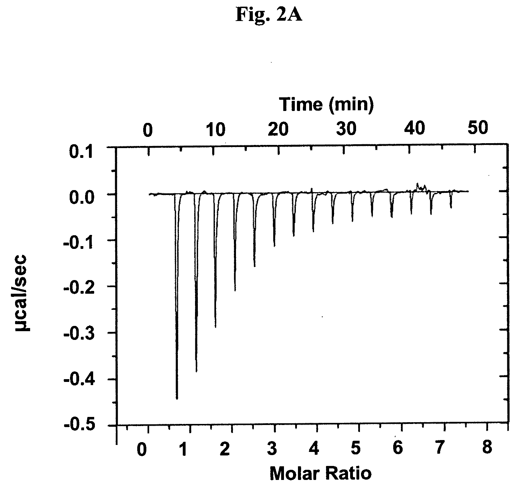

**Figs. 2A-2B** show simulated binding isotherm for the association of PEG₅-MAL-S-(CH₂)₁₅-COOH with HSA. The data was obtained for 15 automatic injections each of 2.7 *µ*l. The total duration of the experiments was 45 min (2A). The concentration of PEG₅-MAL-S-(CH₂)₁₅-COOH in the injection syringe was 400 *µ*M. Sample cell contained HSA at a concentration of 10 *µ*M. Both components were dissolved in PBS-buffer pH=7.4. The experiment was conducted at 23°C. Data: A400HSA10_NDH; model: OneSites; chi^2/DoF=3.454x10⁴; N=1.76±0.0915 sites; K=1.95x10⁵±3.17x10⁴ M⁻¹; ΔH=-1.0374x10⁴±712.6 cal/mol; ΔS=-10.6 (cal/mol)/deg.

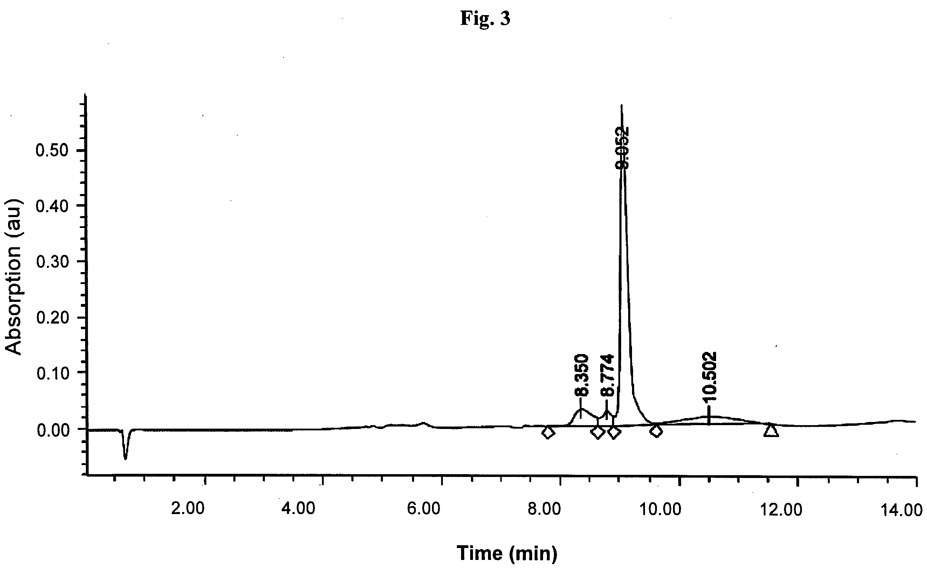

**Fig. 3** shows HPLC analysis of purified insulin-FMS-MAL-S-(CH₂)₁₅-COOH. HPLC-purified insulin-FMS-MAL-S-(CH₂)₁₅-COOH (50 *µ*g) was loaded on a chromolith Rp-18e (100 mm X 4 mm) column and run with a linear gradient from 0 to 100% solution A (0.1% trifluoroacetic acid, TFA) to solution B (acetonitrile-H₂O 75:25 in 0.1% TFA) over 10 min, and then over 4 min in solution B at a rate of 3 ml/min. The effluent was monitored at 220 nm.

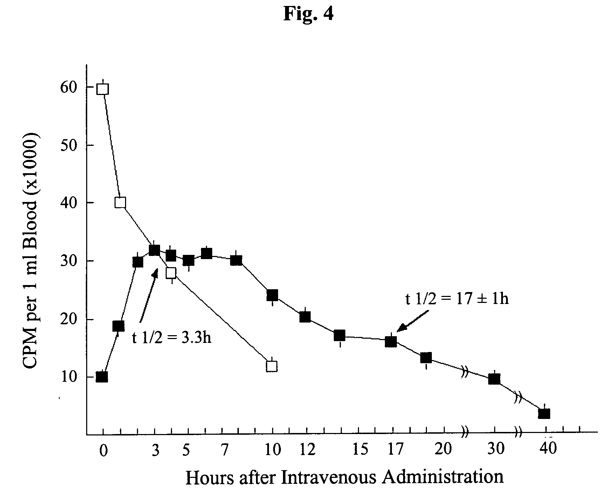

**Fig. 4** shows prolonged residence time of ¹²⁵I-insulin-FMS-MAL-S-(CH₂)₁₅-COOH following intravenous administration in rats. Two groups of rats (n=3 per group) received either ¹²⁵I-insulin (-□-) or HPLC-purified ¹²⁵I-insulin-FMS-MAL-S-(CH₂)₁₅-COOH (-■-; 8.4±0.4 x 10⁶ CPM per 220±10 grams rat). At the indicated time points, blood aliquots (50-70 mg) were drawn and counted for their radioactive content.

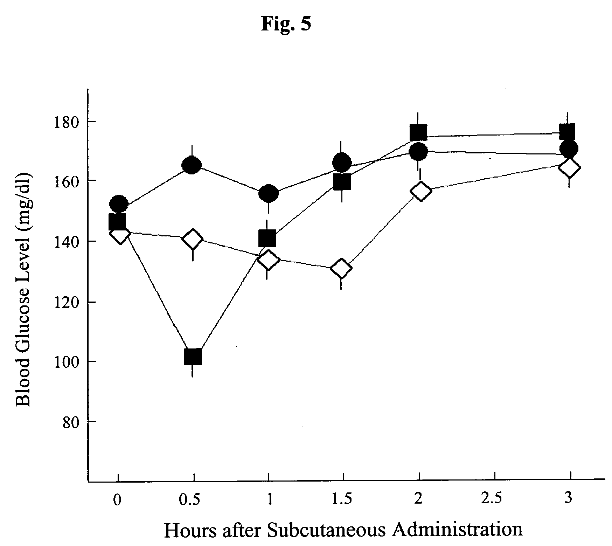

**Fig. 5** shows circulating glucose levels in CD1-mice following a single subcutaneous administration of insulin-FMS-MAL-S-(CH₂)₁₅-COOH. Mice were subcutaneously injected with PBS-buffer (-●-; 0.2 ml/mouse), Zn²⁺ free insulin (-■-; 0.17 nmol/mouse in 0.2 ml PBS buffer) or insulin-FMS-MAL-S-(CH₂)₁₅-COOH (-◊-; 0.17 nmol/mouse in 0.2 ml PBS buffer). Blood glucose levels were determined at the indicated time points. Each point is the arithmetic mean of n=5 mice±SE.

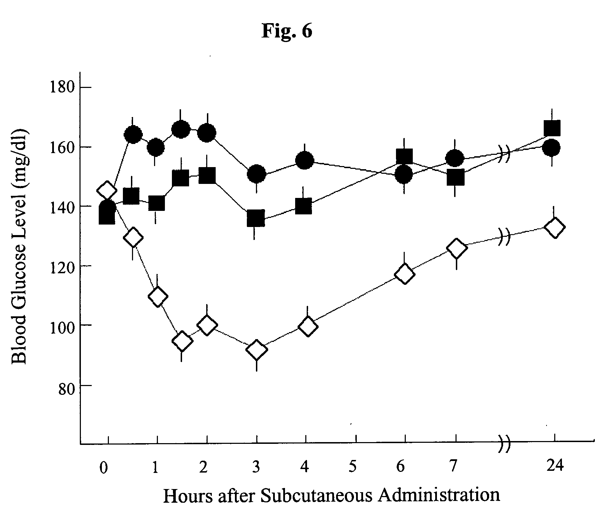

**Fig. 6** shows circulating glucose levels in CD1-mice following a single subcutaneous administration of either insulin-FMS-MAL-S-(CH₂)₁₅-COOH or insulin-detemir. Mice were subcutaneously injected with PBS-buffer (-●-), insulin-FMS-MAL-S-(CH₂)₁₅-COOH (-◊-) or insulin-detemir (-■-; 0.68 nmol/mouse in 0.2 ml PBS buffer), and blood glucose levels were determined at the indicated time points. Each point is the arithmetic mean of n=5 mice±SE.

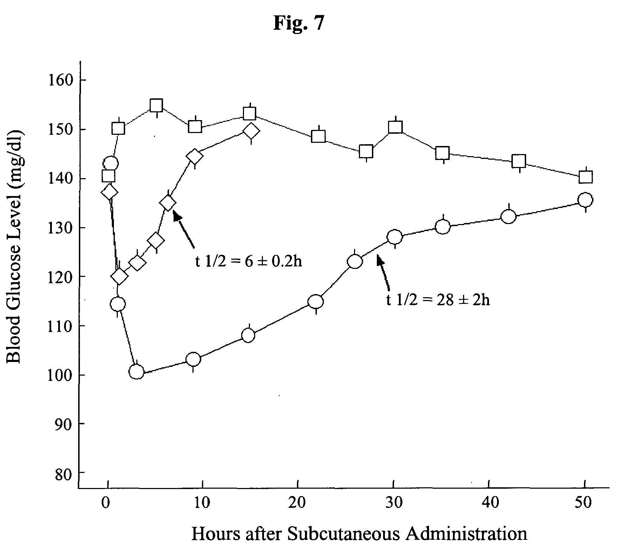

**Fig. 7** shows glucose lowering pattern of exendin-4-FMS-MAL-S-(CH₂)₁₅-COOH, following a single subcutaneous administration to CD1-mice. Three groups of mice (n=6 per group) underwent one subcutaneous administration of PBS buffer (-□-) pH 7.4, native exendin-4 (-◊-; 0.24 nmol/mouse) or exendin-4-FMS-MAL-S-(CH₂)₁₅-COOH (-○-; 0.24 nmol/mouse), and circulating glucose levels were then monitored at the time points indicated in this figure. Each point is the arithmetic mean of n=6 mice±SE.

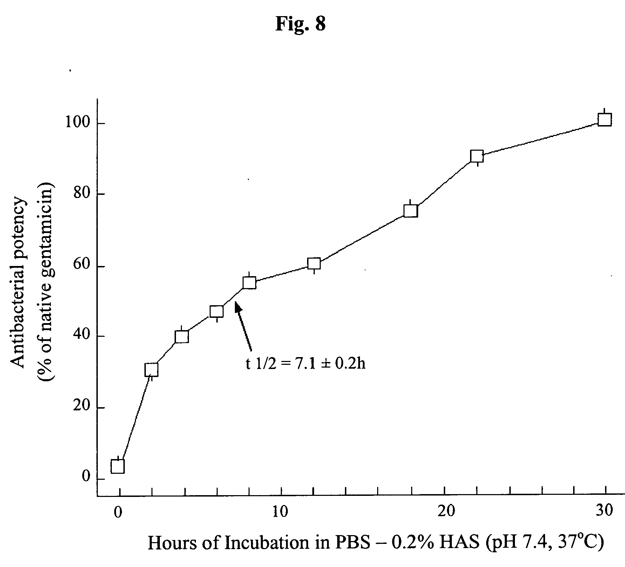

**Fig. 8** shows time course of *in vitro* reactivation of gentamicin-FMS-MAL-S-(CH₂)₁₅-COOH. Gentamicin-FMS-MAL-S-(CH₂)₁₅-COOH (0.16 *µ*moles/ml) was incubated in PBS, pH 7.4, containing 2% (w/v) HSA at 37°C. Aliquots were withdrawn at the indicated time points and analyzed at several concentrations in the antibacterial assay. Gentamicin inhibits *E.Coli* replication with IC₅₀=2.1±0.2 *µ*M. An aliquot with IC₅₀=21±2 *µ*M is considered as having 10% of the native gentamicin antibacterial potency.

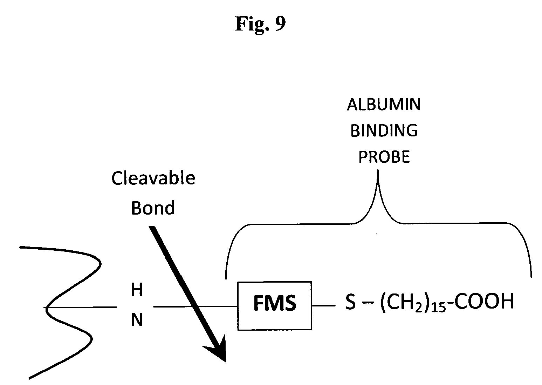

**Fig. 9** shows schematically the principle of converting short-lived drugs into long-lived species *in-vivo* using a certain albumin-binding probe of the present invention. Inactive albumin-associated conjugates bind to serum albumin and therefore exhibit prolonged residence time *in situ,* during which the parent amino containing molecules are released from the inactive conjugates in their native-active form, at a slow rate over many hours following administration.

### MODES FOR CARRYING OUT THE INVENTION

Albumin is the most abundant protein in the blood, at a concentration of approximately 600 µM. One of the physiological roles of albumin is to act as a carrier of fatty acids, due to the fact that long chain fatty acids (LCFAs) bind tightly to albumin, wherein the terminal carboxylate (-CH₂-COOH) serves as an albumin-binding ligand. According to the present invention, this specific property of LCFA like compounds has been exploited in order to associate any amino-containing drug with albumin, thus prolonging the life of said drug in the blood circulation.

In one aspect, the present invention relates to a compound of the formula I, i.e., to an albumin-binding ligand, as defined above.

The term "(C₁-C₈)alkyl", as used herein, typically means a straight or branched hydrocarbon radical having 1-8 carbon atoms and includes, e.g., methyl, ethyl, n-propyl, isopropyl, n-butyl, sec-butyl, isobutyl, tert-butyl, n-pentyl, 2,2-dimethylpropyl, n-hexyl, n-heptyl and n-octyl. The terms "(C₁₃-C₂₀)alkylene" refers to a straight or branched divalent hydrocarbon radical having 13-20 carbon atoms and includes, e.g., n-tridecanylene, n-tetradecanylene, n-pentadecanylene, n-hexadecanylene, n-heptadecanylene, n-octadecanylene, n-nonadecanylene and icosanylene. The terms "(C₁₃-C₂₀)alkenylene" and "(C₁₃-C₂₀)alkynylene" typically mean straight or branched divalent hydrocarbon radicals having 13-20 carbon atoms and one or more double or triple bonds, respectively, wherein each one of said double or triple bonds is not a terminal double or triple bond. Examples of such radicals include 2-, 3-, 4-, 5-and 6-tridecenylene, tetradecenylenes such as myristoleylene, 2-, 3-, 4-, 5-, 6- and 7-pentadecenylene, hexadecenylenes such as palmitoleylene, 2-, 3-, 4-, 5-, 6-, 7-and 8-heptadecenylene, octadecenylenes such as oleylene, linoleylene, α-linoleylene, nonadecenylene, icosenylenes such as arachidonylene and eicosapentylene.

The term "(C₆-C₁₀)aryl" denotes an aromatic carbocyclic group having 6-10 carbon atoms consisting of a single ring or condensed multiple rings such as
phenyl and naphthyl; the term "ar(C₁-C₈)alkyl" denotes an arylalkyl radical such as benzyl and phenetyl; and the term "(C₆-C₁₀)arylene-diyl" denotes a divalent aromatic carbocyclic group having 6-10 carbon atoms consisting of either a single ring or condensed multiple rings such as phenylene and naphthylene.

The term "amino acid residue", as used herein, refers to any natural or synthetic, i.e., non-natural, amino acid residue in its both L- and D-stereoisomers. While a natural amino acid is any one of the twenty amino acid residues typically occurring in proteins, the term synthetic/non-natural amino acid refers to any amino acid, modified amino acid and/or an analog thereof, that is not one of the twenty natural amino acids. The term "aliphatic hydrophobic amino acid residue" refers to an amino acid residue having an aliphatic hydrocarbyl side chain.

Examples of aliphatic hydrophobic amino acids include the natural amino acids leucine, isoleucine and valine, as well as the non-natural amino acids norvaline (Nva), norleucine (Nle), homovaline, and homoleucine. The term "aromatic amino acid residue" refers to an amino acid residue in which the side chain contains an aromatic ring. Examples of aromatic amino acids include the natural amino acid phenylalanine as well as the non-natural amino acids bipyridyl alanine, p-carboxymethyl-L-phenylalanine and p-nitro-L-phenylalanine. The term "amino acid analog comprising -COOH or -SO₃H group" refers to any amino acid analog having amino group as well as -COOH, -SO₃H or both groups, such as taurine.

The term "leaving group", as used herein, refers to any functional group or atom, which can be displaced by another functional group or atom in a substitution reaction, e.g., a nucleophilic substitution reaction. Examples of leaving groups include -O-(CH)₂-CN, 2,5-dioxopyrrolidin-1-olate also known as N-hydroxysuccinimide (herein designated -OSu), 4-nitrophenoxy, 2-nitrophenoxy, 2,3,4,5,6-pentachlorophenoxy, isoindoline-1,3-dione-2-oxy, and benzenesulfanyl, wherein -OSu is preferred.

In certain embodiments, the compound of the present invention is a compound of formula I, wherein R₄ is -SO₃H at position 2 of the fluorene ring, R₅ and R₆ each is hydrogen, and R₇ is -OSu.

In particular embodiments, the compound of the present invention is a compound of formula I, wherein R₄ is -SO₃H at position 2 of the fluorene ring, R₅ and R₆ each is hydrogen, R₇ is -OSu, R₁ is -NH-CO- or -NH-, preferably -NH-CO-, at position 7 of the fluorene ring, R₂ is R₉ or 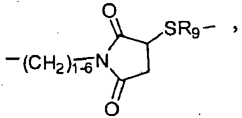 R₉ is selected from (C₁₃-C₂₀)alkylene, (C₁₃-C₂₀)alkenylene or (C₁₃-C₂₀)alkynylene, preferably (C₁₃-C₂₀)alkylene, optionally interrupted by (i) a heteroatom selected from S, O or N, or more than one identical or different heteroatoms selected from S or N, and/or (ii) at least one group selected from -NH-CO-, -CO-NH-, -N(C₁-C₈alkyl)-, -N(C₆-C₁₀aryl)- or -(C₆-C₁₀)arylene-diyl, and R₃ is -COOH or SO₃H. As defined above, it should be noted that in cases wherein the alkylene, alkenylene or alkynylene is interrupted by more than one, e.g., two, heteroatoms, these heteroatoms may be either identical or different heteroatoms, and can be linked sequentially forming, e.g., -S-S- (disulfide) or -N-N- bond, or at any two positions of the alkylene, alkenylene or alkynylene.

In certain particular embodiments, the compound of the present invention is a compound of formula I, wherein R₄ is -SO₃H at position 2 of the fluorene ring, R₅ and R₆ each is hydrogen, R₇ is -OSu, R₁ is -NH-CO- at position 7 of the fluorene ring, R₂ is either R₉ or 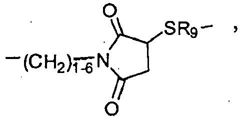 R₉ is (C₁₃-C₂₀)alkylene optionally interrupted by two sulfur atoms forming disulfide bond or by -CO-NH-, and R₃ is -COOH or SO₃H.

The specific compounds of the formula I described in the specification are herein identified by the Arabic numbers **1-8** in bold, wherein their full chemical structures are depicted in **Table 1** hereinafter.

In one specific embodiment, the compound of the present invention is the compound of formula I, wherein R₄ is -SO₃H at position 2 of the fluorene ring, R₅ and R₆ each is hydrogen, R₇ is -OSu, R₁ is -NH-CO-, R₂ is 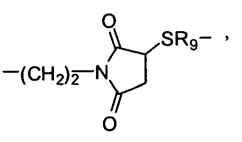 R₃ is -COOH, and R₉ is -(CH₂)₁₅-, i.e., 16-(1-(3-(9-(((2,5-dioxopyrrolidin-1-yloxy)carbonyloxy)-methyl)-7-sulfo-9H-fluoren-2-ylamino)-3-oxopropyl)-2,5-dioxopyrrolidin-3-ylthio) hexadecanoic acid (herein identified SuO-FMS-MAL-S-(CH₂)₁₅-COOH or compound 1).

In another specific embodiment, the compound of the present invention is the compound of formula I, wherein R₄ is -SO₃H at position 2 of the fluorene ring, R₅ and R₆ each is hydrogen, R₇ is -OSu, R₁ is -NH-CO-, R₂ is 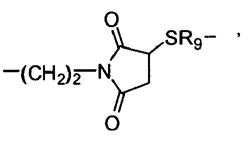 R₃ is -COOH, and R₉ is -(CH₂)₁₅-CO-NH-(CH₂)₅-, i.e., 6-(16-(1-(3-(9-(((2,5-dioxopyrrolidin-1-yloxy)carbonyloxy)methyl)-7-sulfo-9H-fluoren-2-ylamino)-3-oxopropyl)-2,5-dioxopyrrolidin-3-ylthio)hexadecanamido)hexanoic acid (herein identified SuO-FMS-MAL-S-(CH₂)₁₅-CO-N-H-(CH₂)₅-COOH or compound 2).

In a further specific embodiment, the compound of the present invention is the compound of formula I, wherein R₄ is -SO₃H at position 2 of the fluorene ring, R₅ and R₆ each is hydrogen, R₇ is -OSu, R₁ is -NH-CO-, R₂ is 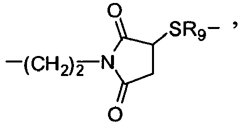 R₃ is -COOH, and R₉ is -(CH₂)₁₀-S-S-(CH₂)₁₀-, i.e., 11-((10-(1-(3-(9-(((2,5-dioxopyrrolidin-1-yloxy)carbonyloxy)methyl)-7-sulfo-9H-fluoren-2-ylamino)-3-oxopropyl)-2,5-dioxopyrrolidin-3-ylthio)decyl)disulfanyl)undecanoic acid (herein identified SuO-FMS-MAL-S-(CH₂)₁₀-S-S-(CH₂)₁₀-COOH or compound 3).

In another specific embodiment, the compound of the present invention is the compound of formula I, wherein R₄ is -SO₃H at position 2 of the fluorene ring, R₅ and R₆ each is hydrogen, R₇ is -OSu, R₁ is -NH-CO-, R₂ is 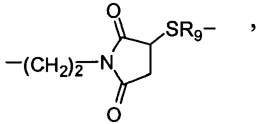 R₃ is -SO₃H, and R₉ is -(CH₂)₁₅-CO-NH-(CH₂)₂-, i.e., 7-(3-(2,5-dioxo-3-(16-oxo-16-(2-sulfoethylamino)hexadecylthio)pyrrolidin-1-yl)propanamido)-9-(((2,5-dioxopyrrolidin-1-yloxy)carbonyloxy)methyl)-9H-fluorene-2-sulfonic acid (herein identified SuO-FMS-MAL-S-(CH₂)₁₅-CO-NH-(CH₂)₂-SO₃H or compound 4).

In still another specific embodiment, the compound of the present invention is the compound of formula I, wherein R₄ is -SO₃H at position 2 of the fluorene ring, R₅ and R₆ each is hydrogen, R₇ is -OSu, R₁ is -NH-CO-, R₂ is R₉, R₃ is - COOH, and R₉ is -(CH₂)₁₅-, i.e., 17-(9-(((2,5-dioxopyrrolidin-1-yloxy)carbonyloxy)methyl)-7-sulfo-9H-fluoren-2-ylamino)-17-oxoheptadecanoic acid (herein identified SuO-FMS-(CH₂)₁₅-COOH or compound **5).**

In yet another specific embodiment, the compound of the present invention is the compound of formula I, wherein R₄ is -SO₃H at position 2 of the fluorene ring, R₅ and R₆ each is hydrogen, R₇ is -OSu, R₁ is -NH-CO-, R₂ is R₉, R₃ is -COOH, and R₉ is -(CH₂)₁₅-CO-NH-(CH₂)₅-, i.e., 6-(17-(9-(((2,5-dioxopyrrolidin-1-yloxy)carbonyloxy)methyl)-7-sulfo-9H-fluoren-2-ylamino)-17-oxoheptadecan-amido)hexanoic acid (herein identified SuO-FMS-(CH₂)₁₅-CO-NH-(CH₂)₅-COOH or compound **6).**

In still a further specific embodiment, the compound of the present invention is the compound of formula I, wherein R₄ is -SO₃H at position 2 of the fluorene ring, R₅ and R₆ each is hydrogen, R₇ is -OSu, R₁ is -NH-CO-, R₂ is R₉, R₃ is-COOH, and R₉ is -(CH₂)₁₀-S-S-(CH₂)₁₀-, i.e., 11-((11-(9-(((2,5-dioxopyrrolidin-1-yloxy)-carbonyloxy)methyl)-7-sulfb-9H-fluoren-2-ylamino)-11-oxoundecyl) disulfanyl)-undecanoic acid (herein identified SuO-FMS-(CH₂)₁₀-S-S-(CH₂)₁₀-COOH or compound 7).

In yet a further specific embodiment, the compound of the present invention is the compound of formula I, wherein R₄ is -SO₃H at position 2 of the fluorene ring, R₅ and R₆ each is hydrogen, R₇ is -OSu, R₁ is -NH-CO-, R₂ is R₉, R₃ is -SO₃H, and R₉ is -(CH₂)₁₅-CO-NH-(CH₂)₂-, i.e., 9-(((2,5-dioxopyrrolidin-1-yloxy) carbonyl oxy)methyl)-7-(17-oxo-17-(2-sulfoethylamino)heptadecanamido)-9H-fluorene-2-sulfonic acid (herein identified SuO-FMS-(CH₂)₁₅- CO-NH-(CH₂)₂-SO₃H or compound 8).

**Table 1: Specific compounds of the invention described in the specification**

| **No**. | **Structure** |
|---|---|
| **1** | 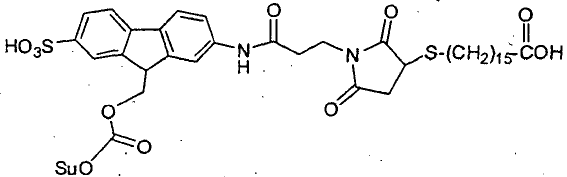 |
| **2** | 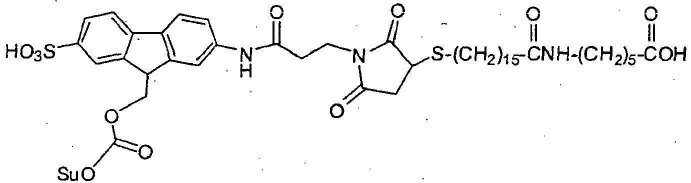 |
| **3** | 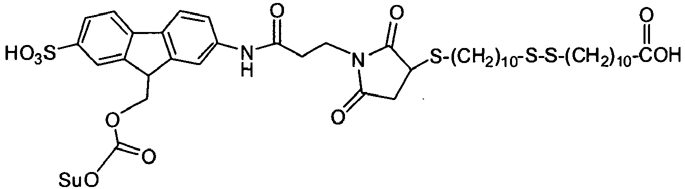 |
| **4** | 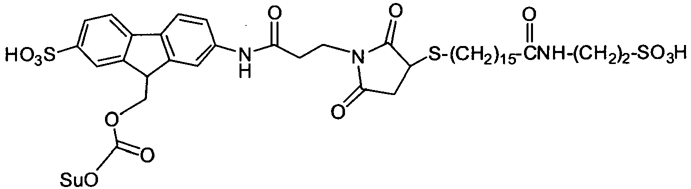 |
| **5** | 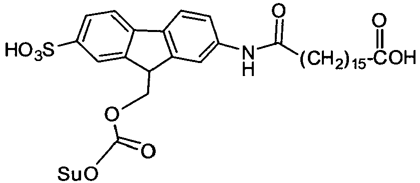 |
| **6** | 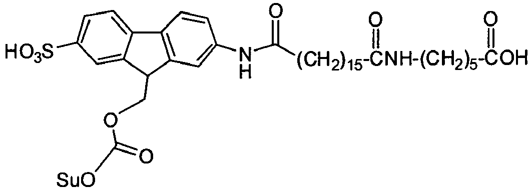 |
| **7** | 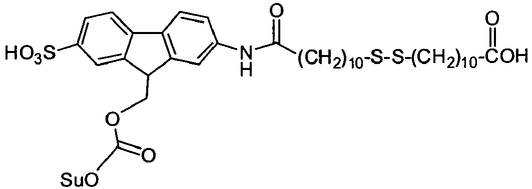 |
| **8** | 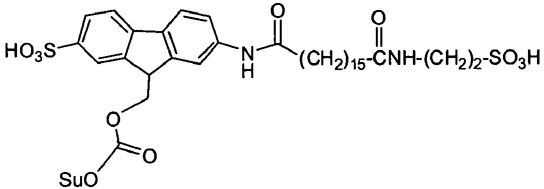 |

The compounds of the present invention may be prepared according to any technology or procedure known in the art, e.g., as described in detail in Tsubery *et al.* (2004) and in various additional publications of the scientific groups of the inventors (Peleg-Shulman *et al.,* 2004; Shechter *et al.,* 2005a; Shechter *et al.,* 2001a; Nesher *et al.,* 2008; Shechter *et al.,* 2007; Shechter, 2005b). For example, the hydrolyzable heterobifunctional intermediate compound 7-(3-(2,5-dioxo-2,5-dihydro-1H-pyrrol-1-yl)propanamido)-9-(((2,5-dioxopyrrolidin-1-yloxy)carbonyl oxy) methyl)-9H-fluorene-3-sulfonic acid, herein designated MAL-FMS-OSu, can be prepared as described in Tsubery *et al.* (2004) starting from 9-hydroxymethyl-2-aminofluorene, and the compounds of the formula I may then be obtained by reacting said MAL-FMS-OSu with, e.g., a HS-(C₁₃-C₂₀)alkylene-COOH, HS-(C₁₃-C₂₀)alkenylene-COOH or HS-(C₁₃-C₂₀)alkynylene-COOH, thus obtaining the corresponding derivatives of the compound of formula I having the general formula SuO-FMS-MAL-S-(C₁₃-C₂₀)alkylene-COOH, SuO-FMS-MAL-S-(C₁₃-C₂₀) alkenylene-COOH or SuO-FMS-MAL-S-(C₁₃-C₂₀)alkynylene-COOH, respectively, which are, in fact, LCFA like molecules containing sulfhydryl moieties, where the terminal carboxylate (-CH₂-COOH) serves as an albumin-binding ligand.

Particular procedures for the preparation of various compounds according to the present invention, in particular, compounds **1, 2, 3** and **4,** are exemplified in Examples 2-5 hereinafter.

The binding properties of the compounds of formula I to human serum albumin (HSA) can be evaluated by any suitable technique, e.g., by isothermal scanning calorimetry (ITC) as exemplified in the Example section hereinafter. As particularly shown in Example 2, both 10-(2,5-dioxopyrrolidin-3-ylthio)decanoic acid and 16-(2,5-dioxopyrrolidin-3-ylthio)hexadecanoic acid, herein designated MAL-S-(CH₂)₁₀-COOH and MAL-S-(CH₂)₁₅-COOH, respectively, were prepared by reacting maleimide moiety with 11-mercaptoundecanoic acid or 16-mercaptohexadecanoic acid, respectively, and associated with HSA yielding Ka values of 1.3 to 1.6x10⁵ M⁻¹. However, while the former completely lost this capability when linked to a 5 kDa polyethylene glycol molecule (PEG₅), i.e., a macromolecule representing a drug, PEG₅-MAL-S-(CH₂))₅-COOH effectively associated with HSA, yielding a Ka value of 1.95x10⁵ M⁻¹ indicating that the length of the LCFA like molecule may significantly influence its ability, when conjugated with a macromolecule such as a drug, to associate with HSA.

Thus, preferred compounds according to the present invention are those in which the shortest chain of atoms linking the fluorene ring and the terminal acidic group associating with albumin, i.e., the hydroxyl group of R₃,
is of 15 to 30 atoms. The term "chain of atoms linking the fluorene ring and the terminal acidic group associating with albumin", as used herein, refers to any chain of atoms formed by the sequence R₁-R₂-R₃ in the compound of formula I, which links the fluorene ring and the hydroxyl group of R₃
through which said compound binds to albumin. In view of the definitions of R₁, R₂ and R₃, said chain of atoms may be interrupted by one or more heteroatoms independently selected from oxygen, nitrogen or sulfur as defined above; functional groups such as -NH-, -NH-CO-, -NH-CO-NH-, -S-, -SO₂NH-, -O-, -OCO-, -CO-NH-, -CS-NH-, -CO-; or a cyclic aliphatic or aromatic ring such as -OSu or phenyl, respectively. Said cyclic aliphatic ring can be linked through any position of the ring, e.g., through positions 1 and 3 in a 5-membered aliphatic ring such as 3-mercaptopyrrolidine-2,5-dione, also known as 3-mercaptosuccineimide (herein designated MAL-S), or through any two positions being located ortho, meta or para one to another, in a 6-membered aromatic ring such as phenyl.

It should be noted that in cases the backbone of R₂ does not contain a cyclic aliphatic or aromatic ring, only one chain of atoms linking the fluorene ring and the terminal acidic group associating with albumin exists. However, in cases a cyclic aliphatic or aromatic ring interrupts the backbone of R₂, e.g., in the case wherein R₁ is -NH-CO-, R₃ is -COOH and R₂ is 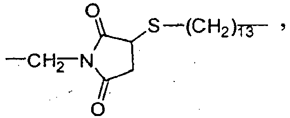 two chains of atoms linking the fluorene ring and the hydroxyl group of R₃ exist, wherein one of said chains consists of the sequence -NH-CO-CH₂-N-CO-CH-S-(CH₂)₁₃-CO-, i.e., is of 21 atoms, and the other chain consists of the sequence -NH-CO-CH₂-N-CO-CH₂-CH-S-(CH₂)₁₃-CO-, i.e., is of 22 atoms. The term "the shortest chain of atoms linking the fluorene ring and the terminal acidic group associating with albumin" thus refers to the chain of atoms linking the fluorene ring and said terminal acidic group in cases a single such chain exists or, alternatively, to the chain of atoms linking the fluorene ring and said acidic group, having the lowest number of atoms in its backbone, in cases more than one such chains exist.

As stated above and shown in the Example section hereinafter, conjugates of formula II formed by introducing a compound of formula I, in particular, SuO-FMS-MAL-S-(CH₂)₁₅-COOH, with various short-lived amino-containing drugs, in particular, insulin, exendin-4, gentamicin, Factor VIIa and Factor VIII, have associating affinity to HSA that is in the range of Ka=2.0-2.6x10⁵ M⁻¹, i.e., sufficient to turn said short-lived drugs to long-lived species *in vivo.* It is expected that corresponding conjugates formed by introducing a compound of formula I having a terminal sulfonyl (-SO₃H) instead of carboxyl, e.g., compounds 4 and 8, with such short-lived amino-containing drugs, would have even higher associating affinity to HSA, due to the higher affinity of the sulfonyl group to HSA compared with that of the carboxyl group.

As commonly known, ligand-protein affinities are affected by non-covalent intermolecular interactions between the two molecules such as hydrogen bonding, electrostatic interactions, hydrophobic and Van der Waals forces, and may also be affected by high concentrations of other macromolecules that cause macromolecular crowding. With this respect, it is particularly known that low molecular-weight drugs suffer a massive loss of pharmacological potency upon conjugation. As further shown herein, gentamicin representing a low molecular-weight drug, when introduced with SuO-FMS-MAL-S-(CH₂)₁₅-COOH to form a conjugate of formula II, regains its full potency upon incubation at physiological conditions.

In another aspect, the present invention thus relates to a conjugate of the formula II as defined above. This conjugate may be obtained by nucleophilic substitution of a compound of the formula I, as defined above, with any amino-containing drug, i.e., by nucleophilic substitution of R₇ in the compound of formula I with the amino group of said drug.

The drug according to the present invention may be any drug containing at least one amino group.

In one embodiment, the drug is an aminoglycoside antibiotic such as gentamicin or amphotericin, an antineoplastic drug such as aminolevulinic acid, or an anthracycline chemotherapeutic agent such as daunorubicin, doxorubicin, epirubicin, idarubicin, mitoxantrone and valrubicin.

In another embodiment, the drug is a peptide or a protein drug of low or medium molecular weight such as insulin, an interferon, preferably IFN-α2, a peptide YY (PYY) agonist, preferably the peptide PYY₃₋₃₆, an exendin, preferably exendin-3 or exendin-4, an exendin analog or exendin agonist, atrial natriuretic peptide (ANP), human growth hormone (hGH), erythropoietin, TNF-α, calcitonin, gonadotropin releasing hormone (GnRH), a GnRH analogue, hirudin, glucagon, a coagulation factor such as Factor VIIa and Factor VIII, and a monoclonal antibody fragment, preferably anti-TNF-α monoclonal antibody fragment.

Insulin is the predominant drug for diabetes mellitus, a group of syndromes characterized by hyperglycemia, altered metabolism of lipids, carbohydrates and proteins, and an increased risk of complications from vascular diseases. Most patients can clinically be classified as having either insulin-dependent (Type I) or insulin-independent diabetes mellitus (Type II). About 90% of diabetic patients in the Western world have Type II diabetes, and about 70% of the Type II diabetics in the United States are also obese, a factor that significantly contributes to insulin resistance. Whereas in Type I diabetes, there is an extensive and selective loss of pancreatic *β*-cells and a state of hypoinsulinemia, there is no significant loss of *β-*cells from the islets in Type II diabetic patients, in which patients the mean plasma concentration of insulin over a 24-hour period is essentially normal or even elevated because of peripheral resistance to the action of the hormone. Nevertheless, individuals with Type II diabetes are relatively insulin deficient, as a normal pancreatic *β*-cell should be capable of secreting amounts of insulin that are considerably greater than normal when confronted with hyperglycemia, thus allowing an individual to maintain euglycemia in the face of moderate resistance to insulin.

Virtually all forms of diabetes mellitus are due to either a decrease in the circulating concentration of insulin (insulin deficiency) or a decrease in response of peripheral tissues to insulin (insulin resistance), in association with an excess of hormones with actions opposite to those of insulin, i.e., glucagon, growth hormone, cortisol and catecholamines.

The half-life of insulin in plasma is about 5-6 min, wherein the degradation of insulin occurs primarily in liver and to a lesser extent in kidney and muscle. Proteolytic degradation of insulin in the liver is primarily receptor mediated. Various modifications have been described in order to create insulin analogs having longer half-lives in the blood circulation, in particular, prodrugs capable of releasing active insulin into the circulation over a relatively long time period, i.e., 8-24 hours, intended to provide the required basal level of insulin for a whole day. One of such long-acting human insulin analogs is the aforesaid insulin detemir (Levemir®, NovoNordisk), produced by a process including expression of recombinant DNA in *Saccharomyces cerevisiae* followed by chemical modification, which said to have up to 24 hours duration of action. In particular, insulin detemir is an insulin analog in which the amino acid threonine in position B30 is omitted and myristic acid has been attached to the amino acid lysine in position B29, i.e., N^{*∈*B29}-tetradecanoyl des(B30) insulin. In the blood, insulin-detemir binds to albumin through the acyl group at position B29 and it is then slowly dissociated from this complex.

As shown in Examples 6-8, a conjugate according to the present invention formed by introducing insulin to a compound of formula I, in particular, the conjugate herein designated insulin-FMS-MAL-S-(CH₂)₁₅-COOH, formed by introducing insulin to SuO-FMS-MAL-S-(CH₂)₁₅-COOH, had about 10% the efficacy of insulin to activate lipogenesis in rat adipocytes yielding an half-maximal effect (ED₅₀) at a concentration of 1.03±0.1 nM; however, it has regained its full lipogenic potency (ED₅₀=0.1±0.02 nM) following 4 hours of incubation under conditions that completely release insulin from the conjugate. Furthermore, while the circulating level of insulin declined yielding a t½ value of 3.3±0.4 hours, the circulating level of said conjugate increased over a period of 2 hours reaching a value of 31,000±1,000 cpm/ml blood, which was stably maintained over a period of 6 hours and than declined with a t½ value of 17±1 hours, and a significant amount, in particular, -10,000 cpm/ml blood, was still evident 30 hours after intravenous administration. In addition, said conjugate had a flat glucose-lowering pattern that was by about two folds prolonged than that of insulin.

The aforesaid conjugate was subcutaneously administered at a dose of 0.68 nmol/mouse and as shown, it was highly potent in reducing blood glucose level over prolong time period with a t½ value of 6±1 hours, wherein low blood glucose level was still evident 24 hours following administration. In particular, although the area under the curve could not accurately integrated, it exceeded five or more times that obtained by similar dose of subcutaneously administered insulin-detemir.

Thus, in certain embodiments, the conjugate of the present invention is obtained by nucleophilic substitution of a compound of the formula I, preferably, any one of compounds 1 to 8, with any of the amino groups of insulin.

Exendins are peptides found in the venom of the Gila-monster, a lizard found in Arizona, and the Mexican Beaded Lizard. Exendin-3 is present in the venom of Heloderma horridum, and exendin-4 is present in the venom of Helodermasuspectum. The exendins have some sequence similarity to several members of the glucagon-like peptide family, with the highest homology, 53%, being to GLP-1[7-36]NH₂, which is also known as proglucagon, and has an insulinotropic effect, stimulating insulin secretion from pancreatic *β*-cells. Exendin-4 is composed of 39 amino acid residues with the carboxy terminus amidated. Exendin-4 potently binds at GLP-1 receptors on insulin-secreting *β*TC1 cells, at dispersed acinar cells from guinea pig pancreas, and at parietal cells from stomach. The use of exendin-3 and exendin-4 as insulinotrophic agents for the treatment of diabetes mellitus and the prevention of hyperglycemia has been previously proposed, e.g., in US 5,424,286.

The glucose-lowering profile of native exendin-4 was compared with that of an exendin-4-based conjugate according to the present invention, in particular, the conjugate herein designated exendin-4-FMS-MAL-S-(CH₂)₁₅-COOH, formed by introducing exendin-4 to SuO-FMS-MAL-S-(CH₂)₁₅-COOH, when subcutaneously administered at a dose of 0.24 nmol/CD1 mouse, and as shown in Example 9, circulating glucose reached its lowest concentration 3 hours following administration of said conjugate and this level was preserved over a period of 20 hours. Returning to initial glucose level took place with a t½ value of 28±2h, which is 4.7 times longer than that obtained by the same dose of the native hormone.

Thus, in other certain embodiments, the conjugate of the present invention is obtained by nucleophilic substitution of a compound of the formula I, preferably, any one of compounds **1** to **8,** with any of the amino groups of exendin-4.

While large protein drugs can accommodate one or two HSA-binding probe(s), each having a size of about 760 daltons, with the preservation of significant amount of their biological/pharmacological potencies (Shechter *et al.,* 2001b; Shechter *et al.,* 2007), low molecular-weight amino containing compounds suffer a massive loss of pharmacological potency upon conjugation, and are thus impractical under these circumstances, unless may be reactivated upon administration. In order to test whether the approach of the present invention may be used for low molecular-weight amino containing compounds as well, a gentamicin-based conjugate according to the present invention, in particular, the conjugate herein designated gentamicin-FMS-MAL-S-(CH₂)₁₅-COOH, formed by introducing gentamicin to SuO-FMS-MAL-S-(CH₂)₁₅-COOH, was used.

Gentamicin is an aminoglycoside antibiotic, used in treatment of many types of bacterial infections, particularly those caused by Gram-negative bacteria. Gentamicin works by binding the 30S subunit of the bacterial ribosome, thus interrupting protein synthesis. As shown in Example 10, the conjugate gentamicin-FMS-MAL-S-(CH₂)₁₅-COOH that was incubated in PBS (pH 7.4) containing 2% (w/v) HSA at 37°C had ∼3±0.7% the antibacterial potency of native gentamicin; however, upon incubation in PBS buffer (pH 7.4) containing 20 mg/ml HSA, the antibacterial potency was generated with a t_{½}, value of 7.1±0.2 h, regaining full (100%) antibacterial potency following 30 hours of incubation.

Thus, in further certain embodiments, the conjugate of the present invention is obtained by nucleophilic substitution of a compound of the formula I, preferably, any one of compounds **1** to **8,** with any of the amino groups of gentamicin.

Factor VII (FVII), formerly known as proconvertin, is a vitamin K dependent enzyme of the serine protease class, produced in the liver, and is one of the central proteins in the coagulation cascade. The main role of FVII is to initiate the process of coagulation in conjunction with tissue factor, which is found on the outside of blood vessels, normally not exposed to the bloodstream. Upon vessel injury, tissue factor is exposed to the blood and circulating FVII. Once bound to tissue factor, FVII is activated to activated FVII (FVIIa) by different proteases, among which are thrombin (Factor IIa), activated Factor X and the FVIIa-tissue factor complex itself. The most important substrates for FVIIa-tissue factor are Factors X (FX) and IX (FIX).

Recombinant human FVIIa has been introduced for use in uncontrollable bleeding in hemophilia patients with Factor VIII (FVIII) or FIX deficiency, who have developed inhibitors against replacement coagulation factor. This factor is increasingly used in uncontrollable hemorrhage, as it induces coagulation only in those sites where tissue factor is present as well. In addition, according to Mayer *et al.* (2005), recombinant human FVII improves outcomes in acute intracerebral hemorrhage.

FVIII is another essential blood clotting factor. In fact, it is a cofactor for activated FIX which, in the presence of Ca⁺² and phospholipids, forms a complex that converts FX to the activated form thereof. In human, FVIII is encoded by the F8 gene, and therefore defects in this gene result in hemophilia A, a common recessive X-linked coagulation disorder. The FVIII gene produces two alternatively spliced transcripts, wherein transcript variant 1 encodes a large glycoprotein, isoform a, which circulates in plasma, associates with von Willebrand factor in a noncovalent complex and undergoes multiple cleavage events, and transcript variant 2 encodes a putative small protein, isoform b, which consists primarily of the phospholipid binding domain of FVIIIc that is essential for coagulant activity.

US 7,199,223 discloses conjugates of a FVIII moiety and one or more water-soluble polymers, each having a molecular weight in the range of 6 to 150 kDa, preferably conjugates wherein each one of the polymers is a poly(alkylene oxide), more preferably a PEG, and the FVIII moiety is either recombinantly produced or blood-derived FVIII, FVIIIa, FVIII:C, FVIII:vWF, and B-domain deleted FVIII. As described in this patent, these conjugates may be used for treating patients in need of FVIII therapy such as patients suffering from hemophilia A. US Publication Nos. 20080058504 and 20090041714, both continuation applications of US 7,199,223, discloses similar conjugates, wherein a water-soluble polymer is covalently attached to the FVIII moiety via either a degradable linkage such as a physiologically hydrolyzable or enzymatically degradable linkage, or a thiol group of a cysteine residue contained within said FVIII moiety.

As described in Example 11, the technology of the present invention may further be applied FVIIa and to FVIII, and the pharmacokinetic pattern of the specific conjugates formed by introducing these coagulation factors to SuO-FMS-MAL-S-(CH₂)₁₅-COOH, i.e., FVIIa-FMS-MAL-S-(CH₂)₁₅-COOH and FVIII-FMS-MAL-S-(CH₂)₁₅-COOH can be studied both *in vitro* as well as in *in vivo* experimental systems. It is expected that both FVIII and FVIIa derivatized with excess of SuO-FMS-MAL-S-(CH₂)₁₅-COOH will have significantly reduced biological activities at time 0 but will undergo reactivation with a t_{½} value of several hours under physiological conditions.

Thus, in still further certain embodiments, the conjugate of the present invention is obtained by nucleophilic substitution of a compound of the formula I, preferably, any one of compounds **1** to **8,** with any of the amino groups of FVIIa or FVIII. It should be noted that both FVIIa and FVIII, according to the present invention, might be either natural or recombinant.

In a further aspect, the present invention provides a pharmaceutical composition comprising a conjugate of the formula II as defined above, or a pharmaceutically acceptable salt thereof, and a pharmaceutically acceptable carrier.

In certain embodiments, the pharmaceutical compositions of the present invention comprise conjugates obtained by nucleophilic substitution of a compound of the formula I, preferably any one of compounds **1** to **8,** with insulin, exendin-4, gentamicin or coagulation factors such as FVIIa and FVIII, or a pharmaceutically acceptable salts thereof.

The pharmaceutical compositions of the present invention may be prepared by conventional techniques, e.g. as described in Remington: The Science and Practice of Pharmacy, 19th Ed., 1995. The composition may be in solid, semisolid or liquid form and may further include pharmaceutically acceptable fillers, carriers or diluents, and other inert ingredients and excipients. Furthermore, the pharmaceutical composition can be designed for a slow release of the conjugate. The composition can be administered by any suitable route, e.g. intravenously, orally, parenterally, rectally, or transdermally. The dosage will depend on the state of the patient, and will be determined as deemed appropriate by the practitioner.

The route of administration may be any route that effectively transports the active compound to the appropriate or desired site of action, the oral and the intravenous routes being preferred. If a solid carrier is used for oral administration, the preparation may be tabletted, placed in a hard gelatin capsule in powder or pellet form or it can be in the form of a lozenge. If a liquid carrier is used, the preparation may be in the form of a syrup, emulsion or soft gelatin capsule. Tablets, dragees or capsules having talc and/or a carbohydrate carrier or binder or the like are particularly suitable for oral application. Preferable carriers for tablets, dragees or capsules include lactose, cornstarch and/or potato starch.

The pharmaceutical compositions of the present invention can be used for treatment of various diseases, disorders or conditions, in which administration of the drug designated Y in the formula II might be useful.

In particular, in still another aspect, the present invention provides a conjugate of formula II, as defined above, obtained by nucleophilic substitution of a compound of formula I, as defined above, preferably, any one of compounds 1 to 8, with any of the amino groups of insulin for use in the treatment of diabetes mellitus or hyperglycemia.

In yet another aspect, the present invention provides a conjugate of formula II, as defined above, obtained by nucleophilic substitution of a compound of formula I, as defined above, preferably any one of compounds 1 to 8, with any of the amino groups of exendin-4 for use in the treatment of insulin-dependent diabetes mellitus, non-insulin-dependent diabetes mellitus, or gestational diabetes mellitus, or for the prevention of hyperglycemia.

In still a further aspect, the present invention provides a conjugate of formula II, as defined above, obtained by nucleophilic substitution of a compound of formula I, as defined above, preferably any one of compounds 1 to 8, with any of the amino groups of gentamicin for use in the treatment of a bacterial infection, preferably a bacterial infection caused by Gram-negative bacteria.

In yet a further aspect, the present invention provides a conjugate of formula II, as defined above, obtained by nucleophilic substitution of a compound of formula I, as defined above, preferably any one of compounds **1** to **8,** with any of the amino groups of FVIIa or FVIII, respectively for use in Factor VIIa or Factor VIII therapy, respectively. In certain embodiments, this conjugate is used for treatment of patients who suffer from hemophilia A.

In summary, the present invention provides a novel technology according to which any amino-containing short-lived drug can be converted, upon administration, into a long-lived prodrug, which gradually releases the pharmacologically active constituent under physiological conditions. This property is particularly achieved by introducing said short-lived drug with a LCFA like molecule capable of associating with HSA *in vivo,* which contains a spontaneously hydrolysable bond.

The strategy disclosed herein has not been materialized before because of two main barriers, i.e., (i) the difficulty to obtain an albumin-binding probe having sufficient associating affinity to albumin, as most small molecules that bind tightly to this carrier protein lose this capability when linked to a macromolecule such as drugs; and (ii) the lack of "reversibility principle" of the type developed in the laboratories of the inventors, which is a prerequisite condition in case the albumin associated conjugate formed *in vivo* has lost its biological/pharmacological potency upon conjugation. It should further be understood that this conjugate-reactivation becomes irrelevant, if does not take place in body fluids at a slow rate with a desirable pharmacokinetic pattern.

The invention will now be illustrated by the following non-limiting Examples.

### EXAMPLES

### Materials and Methods

***(i) Materials.*** Human (Zn²⁺-free) insulin was donated by NovoNordisk (Bagsvalrd, Denemark) or by Biotechnology General (Rehovot, Israel). Insulin-detemir (Levemir®, NovoNordisk) was extensively dialyzed against 0.01 M NaHCO₃ and stored at 7°C until used. The concentration of the insulin-detemir was determined by its absorbance at 280 nm (ε₂₈₀=5900) and/or by acid hydrolyzing an aliquot (in 6 M HCl, for 22 h at 110°C) followed by quantitative amino acid analysis. D-[U-¹⁴C] glucose (4-7 mci/mol) was obtained from Du Pont-NEN (Boston, Ma), type I collagenase (134 U/mg) was purchased from Worthington (Freehold, NY), gentamicin sulfate was purchased from Sigma Chemical Co. (Ness-Ziona, Israel) and polyethylene glycol 5 kDa-maleimide (PEG₅-MAL) was obtained from Shearwater Group Inc. (Ra'anana, Israel). Exendin-4 (HGEGTFTSDLSKQM EEEAVRLFIEWLKNGGPSSGAPPPS-NH₂) was synthesized by the solid phase method using the multiple peptide synthesizer AMS 422 (Abimed Analysen Technik, GmbH). 11-mercapto undecanoic acid, 16-mercaptohexadecanoic acid, decan-1,10-dithiol, taurine and trityl chloride were all purchased from Sigma-Aldrich Ltd. 6-amino-n-hexanoic acid purchased from BDH Ltd. All other materials used were of analytical grade.
(***ii***) ***MAL***-***FMS***-***OSu***, i.e., 7-(3-(2,5-dioxo-2,5-dihydro-1H-pyrrol-1-yl) propanamido)-9-(((2,5-dioxopyrrolidin-1-yloxy)carbonyloxy) methyl)-9H-fluorene-2-sulfonic acid, was synthesized as described in Tsubery *et al.* (2004), starting from 9-hydroxymethyl-2-aminofluorene, and the final product was obtained in 65% yield following four steps of synthesis.
(***iii***) ***PEG₅-NH₂*** is a 5 kDa polyethylene glycol (PEG₅) moiety, in particular, PEG₅-CO-NH-(CH₂)₃-NH₂, and it was prepared by dissolving PEG₅-N-hydroxysuccinimide ester (PEG₅-OSu, Shearwater product) at a concentration of 20 mg/ml in 0.1 M NaHCO₃ containing 1 M of 1,3-diaminopropane dihydrochloride (Aldrich). The reaction was carried out for 1 hour at 25°C, and the product was extensively dialyzed against H₂O, lyophilized and kept at 7°C until used.
(***iv***) ***Isothermal scanning calorimetry measurements*** were performed with iTC₂₀₀ microcalorimeter (MicroCal LLC, Nothampton MA, USA), according to the manufacturer's instructions.
(***v***) ***Iodination of peptides***/***proteins*** using [¹²⁵I] iodine was performed using the chloramine T method (Hunter and Greenwood, 1962). Rat adipocytes were prepared from the fat pads of male Wistar rats (100-200 g) by collagenase digestion (Rodbell, 1964). Lipogenesis, during which [U-¹⁴C] glucose was incorporated into the lipids, was carried out as described by Moody *et al.* (1974).
***(vi) Antibacterial potency*** of gentamicin and derivatives was determined by *E. Coli* replication inhibition. As previously disclosed (Shechter *et al.,* 2002; Marcus *et al.,* 2008), native gentamicin inhibits replication of *E. Coli* (IC₅₀) at a concentration of 2.1±0.2 *µ*M.
(***vii***) ***Blood glucose levels*** were monitored at varying time points following administration of insulin, exendin-4 and derivatives thereof in blood aliquots taken from the tail vein, with a glucose analyzer (Beckman Instruments Fullerton, CA) by the glucose oxidase method. Groups consisted of five or six mice each. Data are presented as means±SE.
*(**viii**) **Radioactive content*** in rat blood following intravenous administration of ¹²⁵I-labeled peptides/proteins was monitored in blood samples taken at varying time points from the tail vein. Blood aliquots (50-70 mg) were absorbed to preweight Whatman 3MM filters that were reweight immediately after immersing blood samples.

### Example 1. Applying isothermal scanning calorimetry for evaluating various ligands-human serum albumin associating affinities

Several versatile procedures were used in the last three decades in order to evaluate the associating affinities of various ligands to albumin (Peters *et al.,* 1996). In this experiment we have applied isothermal scanning calorimetry (ITC), which is a modem approach for determining binding enthalpies, stochiometries and constants for ligand-protein interactions (Chaires, 2008); however, we first wished to confirm that the Ka values for ligand-human serum albumin (HSA) association, provided by the ITC approach, are close to those obtained using other binding strategies.

As stated above, insulin detemir (Levemir®, NovoNordisk) is a long-acting human insulin analog, in which the amino acid threonine in position B30 has been omitted and myristic acid has been attached to the amino acid lysine in position B29, i.e., N^{*ε*B29}-tetradecanoyl des(B30) insulin. In the blood, insulin-detemir binds to HSA through the acyl group at position B29 and it is then slowly dissociated from the complex.

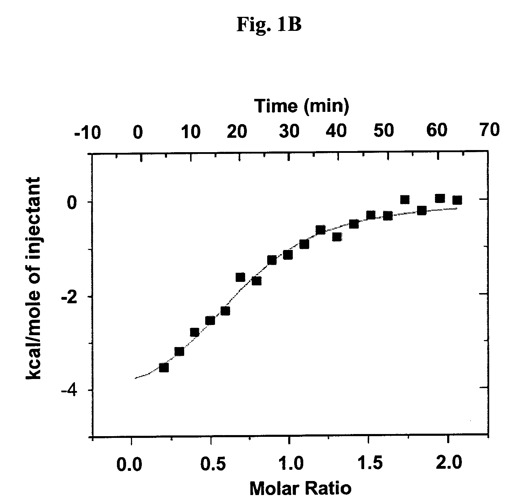

**Figs. 1A-1B** show the binding affinity of insulin-detemir to HSA, as determined by ITC-200. The primary ITC data was translated to a binding isotherm **(****Fig. 1B****)**, providing a binding constant of Ka=0.687±0.16x10⁵ M⁻¹; however, a significantly higher value, i.e., Ka=2.4±0.7x10⁵M⁻¹, was previously obtained for the binding affinity of insulin-detemir to HSA using the immobilized HSA binding strategy (Markusen *et al.,* 1996). Whereas the immobilized HSA binding strategy assumes same binding constants to either immobilized or free albumin (Reed *et al.,* 1975), it appears that ITC yields binding constants that are more accurate than those obtained by partitioning procedures between macromolecules and free ligands (Wiseman *et al.,* 1989). With ITC measurements, ligands or macromolecules "signal" directly the accuracy of the binding process. Spectroscopic signaling can directly reflects associating events as well; however, this depends on the presence of chromophores or fluorophores that alter their properties in the binding state (Wiseman *et al.,* 1989).

### Example 2. Developing a reversible HSA binding probe

In order to design the HSA binding probe, we first searched for long-chain fatty acid (LCFA) like molecules containing sulfhydryl-moieties that can ultimately be linked to the maleimide (MAL) moiety of our hydrolyzable heterobifunctional spacer MAL-FMS-OSu. A required part of any such molecule is a terminal carboxylate (-CH₂-COOH) that is essential for LCFAs' association with albumin (Peters, 1996). Once such compound is found, it should further be evaluated whether it preserves its HSA-associating affinity following its attachment to a macromolecule. As a model for a macromolecule we used PEG₅-maleimide (PEG₅-MAL), i.e., a 5 kDa polyethylene glycol chain that reacts with sulfhydryl containing molecule in a 1:1 stoichiometry. PEG₅-NH₂ was used as well for linking the appropriate HSA binding probe through our hydrolyzable heterobifunctional agent.

### 2.1 Preparation of PEG₅-MAL-S-(CH₂)₁₀-COOH

To a stirred solution of PEG₅-MAL (50 mg in 2 ml H₂O) 2.3 mg of 11-sulfanylundecanoic acid were added (0.1 ml from a fresh solution of HS-(CH₂)₁₀-COOH in dimethylformamide, DMF, 23 mg/ml). After 7 min, 10 mg of solid NaHCO₃ were added. The reaction was carried out for 1 h, and after centrifugation the supernatant was dialyzed overnight against H₂O and lyophilized. The product thus obtained is 5,5'-bisdithio(2-nitrobenzoic acid) (DTNB; Ellman's reagent)-negative.

### 2.2 Preparation of mono modified FMS-MAL-S-(CH₂)₁₅-COOH containing derivatives of insulin and exendin-4.

SuO-FMS-MAL (58.3 mg, 100 µmol) and 16-sulfanylhexadecanoic acid (HS-(CH₂)₁₅-COOH, 38.5 mg, 120 µmol) were dissolved in 1.0 ml DMF, followed by addition of pyridine (20 µl, 248 µmol). The reaction mixture was stirred for 40 min at 25°C, and product formation was monitored by the decrease in the maleimide moiety in aliquots withdrawn during synthesis. Following completion of the reaction (at 4 h) the derivative formed SuO-FMS-MAL-S-(CH₂)₁₅-COOH was added to an aqueous solution of insulin 6 mg/ml (1 µmol/ml) dissolved in 0.1 M NaHCO₃ (pH 8.5) at three molar excess over the protein (30 µl). The reaction was carried out for 2 h at 0°C, and the mixture was then dialyzed against H₂O at 7°C. Monomodified derivative of insulin-linked to FMS-MAL-S-CH₂)₁₅-COOH was purified from un-reacted insulin and from residual bismodified derivative, using semi-preparative HPLC (RP-4 column, Hesperia CA, 20-100% solution B (acetonitrile-H₂O, 75:25 in 0.1% TFA) over 60 min with a flow rate of 10 ml/min). The fraction corresponding to monomodified insulin-FMS-MAL-S-(CH₂)₁₅-COOH was collected, redialyzed against H₂O and lyophilized.

Monomodified derivative of exendin-4 (exendin-4-FMS-MAL-S-(CH₂)₁₅-COOH) was prepared under identical conditions, by adding 3 fold molar excess of the reagent to an aqueous solution of exendin-4 (4.2 mg/ml in 0.1 M NaHCO₃, pH 8.5). The reaction was carried out for 3 h at 0°C, and the reaction mixture was then dialyzed overnight, purified by preparative HPLC, redialyzed and lyophilized.

### 2.3 Discussion

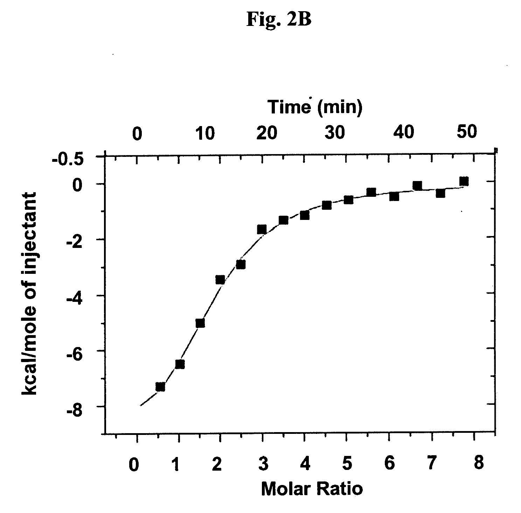

As shown in **Table 2** hereinafter, both 10-(2,5-dioxopyrrolidin-3-ylthio)decanoic acid and 16-(2,5-dioxopyrrolidin-3-ylthio)hexadecanoic acid, prepared by reacting MAL with 11-sulfanylundecanoic acid and 16-sulfanylhexadecanoic acid, and herein designated MAL-S-(CH₂)₁₀-COOH and MAL-S-(CH₂)₁₅-COOH, respectively, associated with HSA yielding Ka values of 1.3 to 1.6x10⁵ M⁻¹. However, while the MAL-S-(CH₂)₁₀-COOH lost the capability to associate with HSA when linked to PEG₅ (PEG₅-MAL-S-(CH₂)₁₀-COOH), the MAL-S-(CH₂)₁₅-COOH linked to PEG₅, i.e., PEG₅-MAL-S-(CH₂)₁₅-COOH, effectively associated with HSA yielding a Ka value of 1.95x10⁵ M⁻¹, as further shown in **Fig. 2B****,** and thus was selected for further designing of the HSA-associating probe.

As further shown in **Table 2,** 2-(((2-(3-(2,5-dioxo-2,5-dihydro-1H-pyrrol-1-yl)propanamido)-7-sulfo-9H-fluoren-9-yl)methoxy)carbonylamino)acetic acid, herein designated glycine-FMS-MAL, associated with HSA with a Ka value of 1.4x10⁵ M⁻¹, but lost its associating affinity towards HSA when linked to a 5 kDa PEG₅-NH₂ chain. This association capacity was regained upon linking HS-(CH₂)₁₅-COOH to the MAL-moiety of the spacer, and as further shown, PEG₅-NH-FMS-MAL-S-(CH₂)₁₅-COOH associated with HSA with a Ka value of 2.57x10⁵ M⁻¹. Similar HSA-associating affinities were obtained when 16-(1-(3-(9-(((2,5-dioxopyrrolidin-1-yloxy)carbonyloxy)methyl)-7-sulfo-9H-fluoren-2-ylamino)-3-oxopropyl)-2,5-dioxopyrrolidin-3-ylthio)hexadecanoic acid, herein designated SuO-FMS-MAL-S-(CH₂)₁₅-COOH was reacted with an amino side chain of either a small peptide, i.e., Gly-His-Lys, or of a larger polypeptide such as exendin-4 (4.2 kDa) or insulin (5.8 kDa), forming Gly-His-Lys-FMS-MAL-S-(CH₂)₁₅-COOH, exendin-4-FMS-MAL-S-(CH₂)₁₅-COOH or insulin-FMS-MAL-S-(CH₂)₁₅-COOH, respectively. It is thus concluded that the covalent introduction of FMS-MAL-S-(CH₂)₁₅-COOH, a specific HSA-binding probe, to peptides/proteins of a variable size, provided them with HSA associating affinity in the range of Ka=2.0-2.6x10⁵ M⁻¹, exceeding about 3.5 times the associating affinity of insulin-detemir to HSA, as shown in **Fig. 1B****.** The structures of some of the compounds and conjugates listed in **Table 2** are shown in Appendix 1.

**Table 2. Binding affinities of various compounds/conjugates to HSA**

| **Compound** | **Association-constant to HSA K M⁻¹** |
|---|---|
| MAL-S-(CH₂)₁₀-COOH | 1.3±0.2x10⁵ |
| MAL-S-(CH₂)₁₅-COOH | 1.55±0.15x10⁵ |
| PEG₅-MAL-S-(CH₂)₁₀-COOH | None |
| PEG₅-MAL-S-(CH₂)₁₅-COOH | 1.95±0.317x10⁵ |
| Glycine-FMS-MAL | 1.4±0.15x10⁵ |
| PEG₅-NH-FMS-MAL | None |
| PEG₅-NH-FMS-MAL-S-(CH₂)₁₅-COOH | 2.57±0.44x10⁵ |
| Gly-His-Lys-FMS-MAL-S-(CH₂)₁₅-COOH | 1.99±0.28x10⁵ |
| Exendin-4-FMS-MAL-S-(CH₂)₁₅-COOH | 2.2±0.2x10⁵ |
| Insulin-FMS-MAL-S-(CH₂)₁₅-COOH | 2.4±0.3x10⁵ |

| | |
|---|---|
| *Simulated binding isotherms for the association of all compounds and conjugates were carried out under the experimental conditions specified in **Fig. 2****.** Each value is the arithmetic mean±SEM of 4-6 determinations. | |

### Example 3. Synthesis of SuO-FMS-MAL-S-(CH₂)₁₅-CO-NH-(CH₂)₅-COOH, 2

### 3.1 Synthesis of trityl-mercaptohexadecanoic acid

A solution of trityl (Tr) chloride (0.53 gr, 1.9 mmol) in dry dichloromethane (DCM, 5 ml) was added dropwise to a solution of 16-mercaptohexadecanoic acid (0.5 gr, 1.7 mmol) and 2,4,6 trimethyl pyridine (452 µl) in dry DCM (5 ml). The mixture was stirred at room temperature under argon for 4 hr. The organic solution was washed with 5% HCl solution and with 2x brine solution, and was then dried with MgSO₄ and evaporated. The reaction was followed by analytical HPLC and thin layer chromatography (TLC). The obtained product was purified by flash chromatography (hexane/ethyl acetate 9:1) receiving 480 mg of trityl-mercaptohexadecanoic acid (53% yield). ¹H NMR (250MHz, CDCl₃) δ: 1.26-1.43 (m, 24H) 1.63-1.68 (m, 2H), 2.15 (t, 2H, J=6 Hz), 2.37 (t, 2H, J=6.25Hz), 7.19-7.32, 7.42-7.45 (m, 15H). Analyzed by mass spectroscopy. [M-H]⁺=530g/mol.

### 3.2 Synthesis of Tr-S-(CH₂)₁₅-CO-NH-(CH₂)₅-COOH

To a stirred solution of trityl-mercaptohexadecanoic acid (200 mg, 0.38 mmol) in dimethylformamide (DMF, 1 ml), N-hydroxysuccinimide (44 mg, 0.38 mmol) and dicyclohexylcarbodiimide (DCC) (376 µl) were added and mixed for 4 hr at room temperature. The reaction vial was centrifuged and the solution was added to 6-amino-n-hexanoic acid (54 mg, 0.4 mmol) in water (0.5 ml) and sodium bicarbonate (pH adjusted to ∼7). The mixture was stirred over night at room temperature. Water were added and the precipitate was centrifuged. This operation was repeated 3 times. The product, Tr-S-(CH₂)₁₅-CO-NH-(CH₂)₅-COOH, was analyzed by analytical HPLC, mass spectroscopy [M-H]⁺=643 g/mol, and ¹H NMR (250 MHz, CDCl₃) δ 1.19-1.69 (m, 30H), 1.95 (m, 2H), 2.15 (m, 2H), 2.35 (m, 2H), 3.26 (m, 2H), 3.45 (m, 2H), 7.21-7.31, 7.41-7.435 (m, 15H).

### 3.3 Synthesis of HS-(CH₂)₁₅-CO-NH-(CH₂)₅-COOH

A solution of Tr-S-(CH₂)₁₅-CO-NH-(CH₂)₅-COOH in dichloromethane/ trifluoroacetic acid/triethyl silane (93:2:5) (5 ml) was stirred for 30 min, and the reaction completion was tested according to the disappearance of the starting material. The solvent was evaporated, producing 140 mg crude HS-(CH₂)₁₅-CO-NH-(CH₂)₅-COOH (yield 91%), which was analyzed by mass spectroscopy [M-H]⁺=400 g/mol.

### 3.4 Synthesis of SuO-FMS-MAL-S-(CH₂)₁₅-CO-NH(CH₂)₅-COOH, 2

SuO-FMS-MAL (40 mg, 0.07 mmol) and HS-(CH₂)₁₅-CO-NH-(CH₂)₅-COOH (85 mg, 0.2 mmol) were mixed in DMF (1 ml) and 2,4,6 trimethyl pyridine (40 µl) for 30 min. The desired product, compound 2, was purified by semi-preparative HPLC (C18 column) and analyzed by mass spectrometry [M-H]⁺=983 g/mol.

### Example 4. Synthesis of SuO-FMS-MAL-S-(CH₂)₁₀-S-S-(CH₂)₁₀-COOH, 3

### 4.1 Synthesis of SuO-FMS-MAL-S-(CH₂)₁₀-SH

SuO-FMS-MAL (25 mg, 4.3×10⁻⁵ mol) and decane-1,10-dithiol (19 µl, 8.8×10⁻⁵ mol) were mixed in DMF (0.3 ml) and 2,4,6 trimethyl pyridine (pH adjusted to ∼7) for 2 hr at room temperature. Purification was done by semi-preparative HPLC (C18 column) receiving 6.3 mg of SuO-FMS-MAL-S-(CH₂)₁₀-SH (18% yield), which was analyzed by mass spectrometry [M-H]⁺=788 g/mol.

### 4.2 Synthesis of undecanoic acid dithio pyridyl

To a stirred solution of 11-mercapto undecanoic acid (10 mg, 4.6×10⁻⁵ mol) in DMF (0.5 ml), 4,4-dithiodipyridyl (50 mg, 0.23 mmol) and 2,4,6-trimethyl pyridine (pH adjusted to -7) were added and mixed for 2 hr at room temperature. The reaction was followed by analytical HPLC. Upon water addition, the product precipitated. After washing 3 times with water and centrifugation, 7.2 mg of undecanoic acid dithio-pyridyl were received (yield 48%), and was then analyzed by mass spectrometry [M-H]⁺=326 g/mol.

### 4.3 Synthesis of SuO-FMS-MAL-S-(CH₂)₁₀-S-S-(CH₂)₁₀-COOH, 3

SuO-FMS-MAL-S-(CH₂)₁₀-SH (3 mg, 3.8×10⁻⁶ mol) and undecanoic acid dithio-pyridyl (2 mg, 5.7×10⁻⁶ mol) are mixed in DMF (0.2 ml) and 2,4,6 trimethyl pyridine (pH adjusted to ∼7) for 2 hr at room temperature, and the desired product, compound 3, is purified using semi-preparative HPLC (C18 column).

Alternatively, in order to obtain compound **3,** one of the sulfanyl groups of 1,11-dithio undecanoic acid is first protected with trytyl, by reacting with trytyl chloride to obtain Tr-S-(CH₂)₁₀-SH, which is then reacted with dithiodipyridyl to obtain Tr-S-(CH₂)₁₀-S-S-pyridyl. Next, Tr-S-(CH₂)₁₀-S-S-pyridyl is reacted with HS-(CH₂)₁₀-COOH to obtain Tr-S-(CH₂)₁₀-S-S-(CH₂)₁₀-COOH, which is then deprotected with DCM, 2% TFA and 5% trimethyl silane, for 30 min, to obtain H-S-(CH₂)₁₀-S-S-(CH₂)₁₀-COOH. Compound **3** is obtained by reacting obtain H-S-(CH₂)₁₀-S-S-(CH₂)₁₀-COOH with the maleimido of the SuO-FMS-MAL spacer.

### Example 5. Synthesis of SuO-FMS-MAL-S-(CH₂)₁₅-CO-NH-(CH₂)₂-SO₃H, 4

The synthesis of SuO-FMS-MAL-S-(CH₂)₁₅-CO-NH-(CH₂)₂-SO₃H was similar to that of compound 2, described in Example 3, by first preparing Tr-S-(CH₂)₁₅CO-NH-(CH₂)₂-SO₃H, which was analyzed by mass spectrometry [M-H]⁺=637 g/mol and HPLC, and then reacting it with SuO-FMS-MAL.

### 5.1 Synthesis of Tr-S-(CH₂)₁₅CO-NH-(CH₂)₂-SO₃H

To a stirred solution of trityl-mercaptohexadecanoic acid (27 mg, 5X10⁻⁵ mol) in DMF (0.5 ml), N-hydroxysuccinimide (7 mg, 6×10⁻⁵ mol) and DCC (40 µl) were added and mixed for 4 hr at room temperature. The reaction vial was centrifuged and the solution was added to taurine (8 mg, 6×10⁻⁵ mol) in water (0.3 ml), resulting in pH of ∼7. The mixture was stirred over night at room temperature. Water was added and the mixture lyophilized. The product, Tr-S-(CH₂)₁₅CO-NH-(CH₂)₂-SO₃H, was analyzed by both analytical HPLC and mass spectroscopy [M-H]⁺=637 g/mol.

### 5.2 Synthesis of HS-(CH₂)₁₅CO-NH-(CH₂)₂SO₃H

The deprotection step of Tr-S-(CH₂)₁₅CO-NH-(CH₂)₂-SO₃H was achieved as described above in Example 3 (section 3.3) for HS-(CH₂)₁₅-CO-NH-(CH₂)₅-COOH, resulting with the crude HS-(CH₂)₁₅CO-NH-(CH₂)₂-SO₃H.

### 5.3 Synthesis of SuO-FMS MAL-S-(CH₂)₁₅-CO-NH-(CH₂)₂-SO₃H, 4

Compound **4** was obtained by reacting the crude HS-(CH₂)₁₅CO-NH-(CH₂)₂-SO₃H with SuO-FMS-MAL, as described above in Example 3 (section 3.4) for compound **2.**

### Example 6. Chemical and biological features of insulin-FMS-MAL-S-(CH₂)₁₅-COOH

Insulin-FMS-MAL-S-(CH₂)₁₅-COOH is a monomodified derivative having molecular-weight of 6570 daltons (calculated value is 6565.5 daltons) as verified by mass spectroscopy. The derivative is soluble in phosphate buffer saline (PBS)-buffer at a concentration of ∼4±1 mg/ml, and it has high absorbance at 280 nm, ε₂₈₀=23,300±1000, amounting to 86% of the calculated value. Insulin-FMS-MAL-S-(CH₂)₁₅-COOH emerged as a single symmetric peak on analytical high-performance liquid chromatography (HPLC)-column with retention time (t_{R}) of 9.052 min, as shown in **Fig. 3****.** Under the same experimental conditions, native insulin elutes with t_{R}=7.28 min.

**Table 3** hereinafter summarizes the characteristic features of HPLC-purified insulin-FMS-(CH₂)₁₅-COOH. As shown, insulin-FMS-MAL-S-(CH₂)₁₅-COOH has about 10% the efficacy of insulin to activate lipogenesis in rat adipocytes yielding an half-maximal effect (ED₅₀) at a concentration of 1.03±0.1 nM. It should be noted, however, that the biological potency of such albumin associated insulin derivative may be significantly reduced, due to the presence of bovine serum albumin (BSA) (10 mg/ml) in this particular assay, as previously noted with insulin-detemir (data not shown). As further shown in **Table 3,** insulin-FMS-MAL-S-(CH₂)₁₅-COOH has regained its full lipogenic potency (ED₅₀=0.1±0.02 nM) following 4 hours of incubation at pH 10.3 and 25°C, i.e., under conditions that completely release insulin from the conjugate (Shechter *et al.,* 2005b).

**Table 3. Chemical and biological features of insulin-FMS-MAL-S-(CH₂)₁₅-COOH**

| | |
|---|---|
| MS (m/z) calculated | 6566 daltons |
| MS (m/z)*^{(a )}*found | 6570 daltons |
| Solubility in aqueous buffer pH 7.4 | 4±1 mg/ml |
| Molar extinction coefficient | |
| calculated ^{(b)} | ε₂₈₀=26,800 |
| found ^{(c)} | ε₂₈₀=23,30±1000 |
| HPLC analysis retention time ^{(d)} | 9.052 min |
| Lipogenic potency in rat adipocytes | End50=1.03±0.1 nM (∼10%) |
| (pH 10.3, 4h, 25°C) | End50=0.1±0.02 nM (∼100%) |

| | |
|---|---|
| ^{(a)} Mass spectra were determined using electrospray single quatropole mass spectroscopy (ESMS). ^{(b)} Was calculated by combining the ε₂₈₀ values for insulin (ε₂₈₀=5800) and MAL-FMS-OSu (ε₂₈₀=21,200). ^{(c)} Was determined by hydrolyzing an aliquot in 6 M HCl (110 °C, 22h) followed by quantitative amino-acid analysis. ^{(d)} Analytical HPLC-analysis was carried out under the experimental conditions described in Materials and Methods. Under these conditions, insulin elutes with t_{R}=7.28 min and has a surface area of 240,600±8,000 mav/*µ*g of insulin. | |

### Example 7. Insulin-FMS-MAL-S-(CH₂)₁₅-COOH has prolonged life time following intravenous administration to rats

In this experiment, two groups of rats (n=3 per group) were intravenously administered with either ¹²⁵I-insulin or HPLC-purified ¹²⁵I-insulin-FMS-MAL-S-(CH₂)₁₅-COOH (8.4±0.4x10⁶ counts per minute (CPM) per 220±10 grams rat), and at indicated time points, blood aliquots (50-70 mg) were drawn and counted for their radioactive content in cpm/ml blood. As shown in **Fig. 4****,** the circulating level of radioactive-labeled-insulin declined yielding a t½ value of 3.3±0.4 h; while the circulating level of radioactive-labeled-insulin-FMS- MAL-S-(CH₂)₁₅-COOH increased over a period of two hours reaching a value of 31,000±1,000 cpm/ml blood, which was stably maintained over a period of 6 hours and than declined with a t½ value of 17±1 hours. A significant amount, in particular, -10,000 cpm/ml blood, was still evident 30 hours after intravenous administration.

### Example 8. Glucose lowering pattern in mice following subcutaneous administration of insulin-FMS-MAL-S-(CH₂)₁₅-COOH

In this experiment, the glucose-lowering pattern obtained after a single subcutaneous administration of insulin-FMS-MAL-S-(CH₂)₁₅-COOH was compared with that of Zn²⁺-free insulin, both administered at a low and similar dose (0.17 nmol/mouse in 0.2 ml PBS buffer). As shown in Fig. 5, insulin-FMS-MAL-S-(CH₂)₁₅-COOH had a flat glucose-lowering pattern that was about two folds prolonged than that of insulin. Interestingly, the area under the curve of insulin-FMS-MAL-S-(CH₂)₁₅-COOH resembled that of the native hormone, although the former has, *in vitro,* only 10% the biological potency of insulin, as shown in **Table 3** hereinabove. Thus, in terms of units/mg, insulin-FMS-MAL-S-(CH₂)₁₅-COOH equals native insulin.

It should be noted that this glucose lowering pattern was previously determined for new insulin preparations in rabbits (Kahn and Shechter, 1990); however, we do not recommend doing so in this species with an insulin derivative that associates with albumin, as rabbit albumin exhibits extraordinarily high affinity to fatty-acid acylated insulins (Kurtzhals *et al.,* 1996).

As shown in **Figs. 1A-1B** and **Table 2,** utilizing ITC measurements it was revealed that insulin-FMS-MAL-S-(CH₂)₁₅-COOH has a binding affinity to HSA that is 3.5 times higher than that of insulin-detemir, i.e., N^{*∈*B29}-tetradecanoyl des(B30) insulin. **Fig. 6** shows the glucose lowering pattern of insulin-FMS-MAL-S-(CH₂)₁₅-COOH as compared to that of insulin-detemir, when subcutaneously administered at a dose of 0.68 nmol/mouse. At this dose, insulin-FMS-MAL-S-(CH₂)₁₅-COOH was highly potent in reducing blood glucose level and it did so over prolong period with a t½ value of 6±1 hours. Low blood glucose level was still evident 24 hours following administration. The area under the curve could not, therefore, be accurately integrated; however, it exceeded five or more times that obtained by similar dose of subcutaneously administered insulin-detemir. As described in Shechter *et al.* (2003), subcutaneous administration of Zn²⁺-free insulin to mice at this dose is severely hypoglycemic.

### Example 9. Exendin-4-FMS-MAL-S-(CH₂)₁₅-COOH facilitates prolonged glucose-lowering effect in CD1 mice

As previously described (Tsubery *et al.,* 2004; Shechter *et al.,* 2003), the CD1 strain of mice reflects well the action of exendin-4, a glucagon-like peptide-1 agonist, in healthy and in type II diabetic patients, in the sense that at any dosage applied, circulating blood glucose level never falls below a threshold level which in CD1-mice amounts to a decrease of 27±3%.

**Fig. 7** shows the glucose-lowering profile of native exendin-4 *vs*. exendin-4-FMS-MAL-S-(CH₂)₁₅-COOH, both subcutaneously administered at a dose of 0.24 nmol/CD 1 mouse. As particularly shown, following administration of exendin-4-FMS-MAL-S-(CH₂)₁₅-COOH, circulating glucose reached its lowest concentration 3 hours after administration and this level was preserved over a period of 20 hours. Returning to initial glucose level took place with a t½ value of 28±2 hours, which is 4.7 times longer than that obtained by the same dose of the native hormone.

### Example 10. Gentamicin-FMS-MAL-S-(CH₂)₁₅-COOH is an inactive-reactivable prodrug

While large protein drugs can accommodate one or two HSA-binding probe(s), each having a size of about 760 daltons, with the preservation of significant amount of their biological/pharmacological potencies (Shechter *et al.,* 2001b; Shechter *et al.,* 2007), low molecular-weight amino containing compounds suffer a massive loss of pharmacological potency upon conjugation. Under these circumstances, low molecular-weight compounds are thus impractical, unless may be reactivated upon administration.

**Fig. 8** shows time course of *in vitro* reactivation of gentamicin-FMS-MAL-S-(CH₂)₁₅-COOH. In particular, gentamicin-FMS-MAL-S-(CH₂)₁₅-COOH (0.16 *µ*moles/ml) was incubated in PBS, pH 7.4, containing 2% (w/v) HSA at 37°C, and aliquots were withdrawn at certain time points and analyzed at several concentrations in the antibacterial assay. As particularly shown, at time 0, gentamicin-FMS-MAL-S-(CH₂)₁₅-COOH had ∼3±0.7% the antibacterial potency of native gentamicin; however, upon incubation in PBS buffer (pH 7.4) containing 20 mg/ml HSA, the antibacterial potency of this conjugate was generated with a t½ value of 7.1±0.2 hours, regaining full (100%) antibacterial potency following 30 hours of incubation.

### Example 11. Factor VIIa and Factor VIII derivatized with OSu-FMS-MAL-S-(CH₂)₁₅-COOH, 1

In this experiment, the technology described in the Examples above is applied to recombinant Factor VIII (rFVIII), an essential protein for normal blood coagulation, and to recombinant Factor VIIa (rFVIIa), a protease that participates in the clotting cascade as well. In particular, each of these two proteins is treated with 2 to 10 molar excess of SuO-FMS-MAL-S-(CH₂)₁₅-COOH, obtaining derivatives having low biological potencies of the native proteins, which are expected to regain 70-90% biological activity upon incubation at physiological conditions.

Derivatization of the proteins is carried out in 0.1 M Hepes (pH 7.4) containing 1 mg/ml of rFVIII or rFVIIa. SuO-FMS-MAL-S-(CH₂)₁₅-COOH is added at 2 to 10 molar excess. Proteins are then examined for their biological potency prior to (time 0) and following their incubation at 37°C in 0.1 M Hepes pH 7.4, containing 140 mM NaCl and 20 mg/ml BSA. Native rFVIII and its derivative are diluted to a final concentration of 1 ng/ml, prior of being assayed, and their biological potencies are estimated with a Coatest-SP4 FVIII (Chromogenix) kit. Biological potencies of rFVIIa and its derivative are obtained by the clotting assay using "Activated Factor VIIa, STACLOT® VIIa-Rtf" (Agis, Bnei-Brak, Israel).

It is expected that both rFVIII and rFVIIa derivatized with excess of SuO-FMS-MAL-S-(CH₂)₁₅-COOH will have a significantly reduced biological activity at time 0 but will undergo reactivation with a tl/2 value of several hours under physiological conditions. The pharmacokinetic patterns of these derivatives can be studied in *in vivo* experimental systems such as increased half-life after derivatives administration in FVIII-deficient mice or estimating FVIIa activity in ret serum following FVII derivative administration in rats, and it is expected that such derivatives will show considerable half-life prolongation, due to their capability to associate with serum albumin, to thereby substantially decrease their clearing rates *in situ.*

### APPENDIX

| **Name** | **Structure** |
|---|---|
| MAL-S-(CH₂)₁₅-COOH | 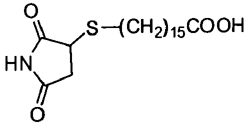 |
| PEG₅-MAL-S-(CH₂)₁₅-COOH | 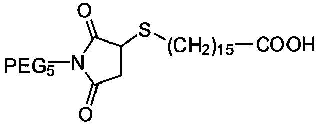 |
| Glycine-FMS-MAL | 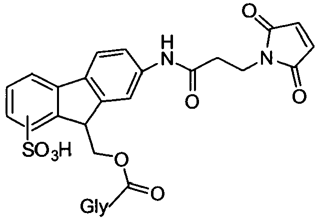 |
| PEG₅- FMS-MAL | 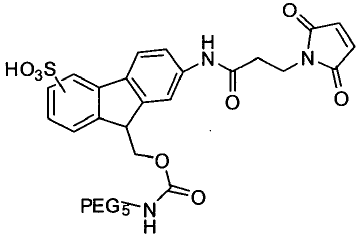 |
| Gly-His-Lys-FMS-MAL-S-(CH₂)₁₅-COOH | 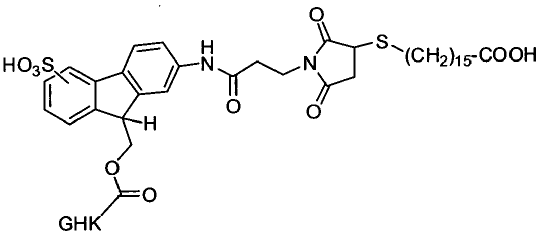 |
| Exendin-4-FMS-MAL-S-(CH₂)₁₅-COOH | 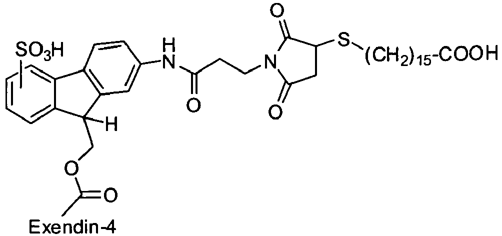 |
| Insulin-FMS-MAL-S-(CH₂)₁₅-COOH | 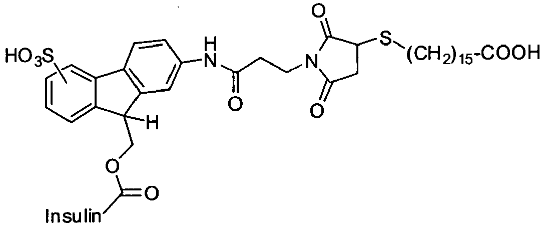 |
| Factor VIIa-FMS-MAL-S-(CH₂)₁₅-COOH | 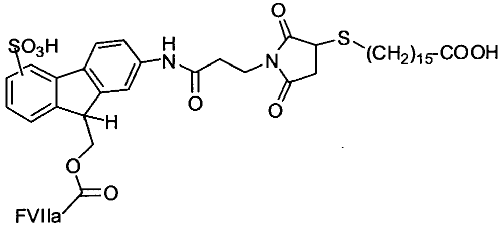 |
| Factor VIII-FMS-MAL-S-(CH₂)₁₅-COOH | 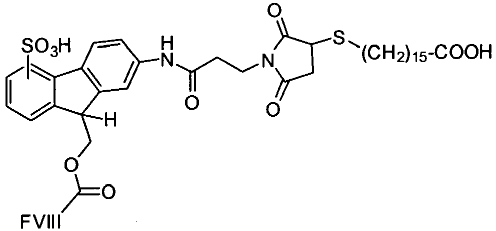 |

### REFERENCES

Bailon P., Palleroni A., Schaffer C.A., Spence C.L., Fung W.J., Porter J.E., Ehrlich G.K., Pan W., Xu Z.X., Modi M.W., Farid A., Graves m., Rational design of a potent, long-lasting form of interferon: a 40 kDa branched polyethylene glycol-conjugated interferon alpha-2a for the treatment of hepatitis C, Bioconjug Chem., 2001, 12, 195-202
Carter D.C., Ho J.X., Structure of serum albumin. In advances in Protein Chemistry (Schumaker, V. N., Ed.), 1994, 153-203, Academic Press, Inc., San Diego, CA
Chaires J.B., Calorimetry and Thermodynamics in Drug Design. Ann. Rev. Biophys., 2008, 37, 135-151
Clark R., Olson K., Fuh G., Marian M., Mortensen D., Teshima G., Chang S., Chu H., Mukku V., Canova-Davis E., Somers T., Cronin M., Winkler M., Wells J.A., Long-acting growth hormones produced by conjugation with polyethylene glycol, J Biol Chem., 1996, 271, 21969-21977
Delgado C., Sancho P., Mendieta J., Luque J., Ligand-receptor interactions in affinity cell partitioning. Studies with transferrin covalently linked to monomethoxypoly(ethylene glycol) and rat reticulocytes, J Chromatogr., 1992, 594,97-103
Goodman L., Gilman A.G., The Pharmacological Basis of Therapeutics, McGraw-Hill, New York, 1995
Hunter W.M., Greenwood F.C., Preparation of iodine-131 labeled human growth hormone of high specific activity, Nature, 1962, 194, 495-496
Kahn C.R., Shechter Y., Insulin, oral hypoglycemic agents and pharmacology of the endocrine pancreas, in A.G. Gilman, T.W. Rall, A.S. Nies and P. Taylor (eds.), Goodman and Gilman Handbook of Pharmacology, New York/Oxford, Pergamon Press, 1990, 1463-1495
Kurtzhals P., Haveland S., Jonassen I., Kiehr B., Larsen U.D., Ribel V., Markussen J., Albumin binding of insulins acylated with fatty acids: Characterization of the ligand-protein interaction and correlation between binding affinity and timing of the insulin effect in vivo, Biochemical J., 1995, 312, 725-731
Kurtzhals P., Haveland S., Jonassen I., Kiehr B., Ribel U., Markussen J., Albumin binding and time action of acylated insulins in various species. J. Pharmaceut. Sci., 1996, 85, 304-308
Kurtzhals P., Haveland S., Jonassen I.B., Markussen J., Effect of fatty acids and selected drugs on the albumin binding of a long-acting, acylated insulin analogue, J. Pharmaceut. Sci., 1997, 86, 1365-1368
Marcus Y., Sasson K., Fridkin M., Shechter Y., Turning low-molecular-weight drugs into prolonged acting prodrugs by reversible pegylation: a study with gentamicin, J. Med. Chem., 2008, 51, 4300-4305
Markusen J., Havelund S., Kurtzhals P., Andersen A.S., Halstrøm E., Hasselager E., Larsen V.D., Ribbel U., Schafter L., Jonassen V.I., Soluble, fatty acid acylated insulins bind to albumin and show protracted action in pigs, Diabetologia, 1996, 39, 281-288
Mayer S., Brun N., Begtrup K., Broderick J., Davis S., Diringer M., Skolnick B., Steiner T., Recombinant activated factor VII for acute intracerebral hemorrhage, N. Engl. J. Med., 2005, 352, 777-785
Moody A.J., Stan M., Gliemann J., A simple free fat cell bioassay for insulin, Horm. Metab. Res., 1974, 6, 12-16
Nesher M., Vachutinsky Y., Fridkin G., Schwarz Y., Sasson K., Fridkin M., Shechter Y., Lichtstein D., Reversible pegylation prolongs the hypotensive effect of atrial natriuretic peptide. Biochonjugate Chem., 2008, 19, 342-348
Peleg-Shulman T., Tsubery H., Mironchik M., Fridkin M., Schreiber G., Shechter Y., reversible PEGylation: A novel technology to release native interferon alpha2 over a prolonged time period, J. Med. Chem., 2004, 47, 4897-4904
Peters T.J., The albumin molecule: Its structure and chemical properties. All about albumin, Biochemistry, Genetics and medical applications, 1996, 24-54, Academic Press, Inc., San Diego, CA
Reddy K.R., Controlled-release, pegylation, liposomal formulations: new mechanisms in the delivery of injectable drugs, Ann Pharmacother., 2000, 34, 915-923
Reed R.G., Gates T., Peters T.Jn., Albumin immobilizing on agarose as a tool for measuring ligand binding of proteins or peptides, Anal. Biochem., 1975, 69, 361-371
Rodbell M., Metabolism of isolated fat cells: effects of hormones on glucose metabolism and lipolysis, J. Biol. Chem., 1964, 239, 375-380
Shechter Y., Goldwaser I., Lavon I., Gershonov E., Mester B., Mironchik M., Patt L.P., Fridkin M., A new approach for prolonging the half-life of peptides, proteins and low-molecular-weigh drugs in vivo, Drugs of the Future, 2001a, 26, 669-676
Shechter Y., Patt L., Schreiber G., Fridkin M., Prolonging the half-life of human interferone-α2 In circulation: Design, preparation, and analysis of FMS7-interferone-α2. Proc. Natl. Acad. Sci. USA, 2001b, 98, 1212-1217
Shechter Y., Tsubery H., Fridkin M., N-[(2-sulfo)-9-fluorenylmethoxy carbonyl)3-gentamicin C1 is a long-acting prodrug derivative, J. Med. Chem, 2002, 45, 4264-4270
Shechter Y., Tsubery H., Fridkin M., Suspensions of pro-drug insulin greatly prolong normoglycemic patterns in diabetic rats, Biochem Biophys Res Commun., 2003, 307, 315-321
Shechter Y., Tsubery H., Mironchik M., Rubinstein M., Fridkin M., Reversible PEGylation of peptide YY3-36 prolongs its inhibition of food intake in mice, FEBS Letts., 2005a, 579, 2439-2444
Shechter Y., Morinchik M., Rubinraut S., Saul A., Tsubery H., Fridkin M., Albumin-insulin conjugate releasing insulin slowly under physiological conditions: A new concept for long-acting insulin, Bioconjugate Chem., 2005b, 16, 913-920
Shechter Y., Mironchik M., Saul A., Gershonov E., Patt L.P., Sasson K., Tsubery H., Mester B., Kapitokovsky A., Rubinraut S., Vachutinski Y., Fridkin G., Fridkin M., New technologies to prolong life-time of peptide and protein drugs in vivo, Intl. J. Peptide Res. and Therapeutics, 2007, 13, 105-117
Shechter Y., Morinchik M., Rubinraut S., Tsubery H., Sasson K., Marcus Y., Fridkin M., Reversible pegylation of insulin facilitates its prolonged action in vivo, Eur. J. Pharm. Biopharm., 2008, In press
Taylor A., Granger D.N., Exchange of macromolecules across the microcirculation, In Handbook of Physiology. E.M. Renkin and C.C. Michel, editors. American Physiological Society, Bethesda, 1984, 467-520
Tsubery H., Mironchik M., Fridkin M., Shechter Y., Prolonging the action of protein and peptide drugs by a novel approach of reversible polyethylene glycol modification, J. Biol. Chem., 2004, 279, 38118-38124
Wiseman T., Williston S., Brandts J.F., Nan Lin Lung, Rapid measurements of binding constants and heats of binding using a new titration calorimeter, Analytical Biochemistry, 1989, 179, 131-137

## Claims

1. A compound of the formula I: 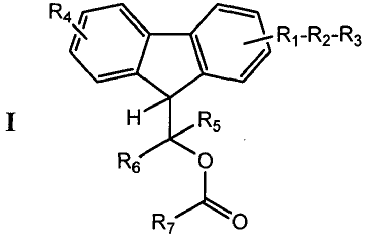 wherein
R₁ is selected from -NH-, -NH-CO-, -NH-CO-NH-, -S-, -SO₂NH-, -O-, - OCO-, -CO-NH-, -CS-NH-, -CO(CH₂)₁₋₄-, or -R₈-CO-, wherein R₈ is (C₁-C₈)alkyl optionally interrupted by a heteroatom selected from O, S or N;
R₂ is selected from 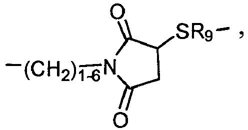 or R₉;
R₃ is an acidic group having at least one hydroxyl group such as -COOH, - SO₃H or -O-PO₃H₂;
R₄ is an electron withdrawing group such as -SO₃H, -CN, -CO-(C₁-C₈)alkyl, -CO-(C₆-C₁₀)aryl, -NO₂, -OPO₃H₂, -N(R)₃⁺, -SO₂NH₂, or halogen, wherein R is selected from (C₁-C₈)alkyl or (C₆-C₁₀)ar(C₁-C₈)alkyl;
R₅ and R₆, each independently is selected from hydrogen, -(C₁-C₈)alkyl or - (C₆-C₁₀)aryl;
R₇ is a leaving group such as -O-(CH₂)₂-CN, -Cl, 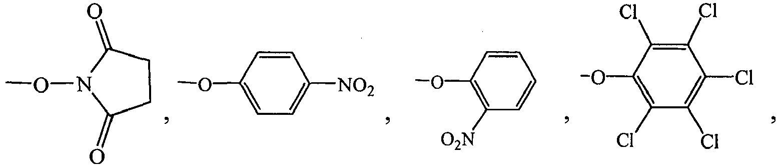 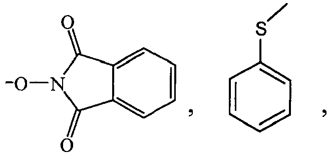 and 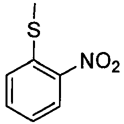 ; and R₉ is selected from (C₁₃-C₂₀)alkylene, (C₁₃-C₂₀)alkenylene or (C₁₃-C₂₀)alkynylene, optionally interrupted by (i) a heteroatom selected from S, O or N, or more than one identical or different heteroatoms selected from S or N, and/or (ii) at least one group selected from -NH-CO-, -CO-NH-, -N(C₁-C₈alkyl)-, -N(C₆-C₁₀aryl)-, or -(C₆-C₁₀)arylene-diyl-, wherein said alkenylene or alkynylene comprises one or more double or triple bond, respectively, and said one or more double or triple bond is not a terminal double or triple bond.

2. The compound of claim 1, wherein R₄ is -SO₃H at position 2 of the fluorene ring, R₅ and R₆ each is hydrogen, and R₇ is N-hydroxysuccinimide (-OSu).

3. The compound of claim 2, wherein R₁ is -NH-CO- or -NH- at position 7 of the fluorene ring, R₂ is R₉ or 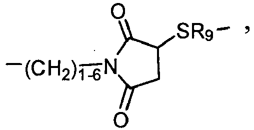 R₉ is selected from (C₁₃-C₂₀)alkylene, (C₁₃-C₂₀)alkenylene or (C₁₃-C₂₀)alkynylene, optionally interrupted by (i) a heteroatom selected from S, O or N, or more than one identical or different heteroatoms selected from S or N, and/or (ii) at least one group selected from -NH-CO-, -CO-NH-, -N(C₁-C₈alkyl)-, -N(C₆-C₁₀aryl)- or -(C₆-C₁₀)arylene-diyl-, and R₃ is -COOH or -SO₃H.

4. The compound of claim 3, wherein R₁ is -NH-CO-, R₂ is R₉ or 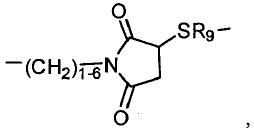 and R₉ is (C₁₃-C₂₀)alkylene, (C₁₃-C₂₀)alkenylene or (C₁₃-C₂₀)alkynylene, preferably (C₁₃-C₂₀)alkylene, optionally interrupted by (i) a heteroatom selected from S, O or N, or more than one identical or different heteroatoms selected from S or N, and/or (ii) at least one group selected from -NH-CO-, -CO-NH-, -N(C₁-C₈alkyl)-, -N(C₆-C₁₀aryl)- or -(C₆-C₁₀)arylene-diyl-.

5. The compound of claim 4, wherein R₂ is 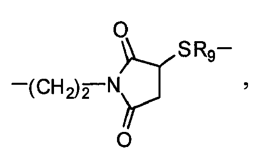 and:
(i) R₃ is -COOH, and R₉ is -(CH₂)₁₅- (herein identified compound 1), of the formula: 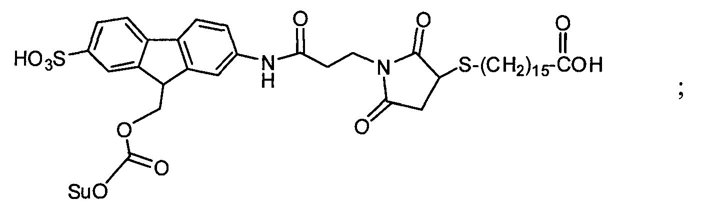
(ii) R₃ is -COOH, and R₉ is -(CH₂)₁₅-CO-NH-(CH₂)₅- group (herein identified compound 2), of the formula: 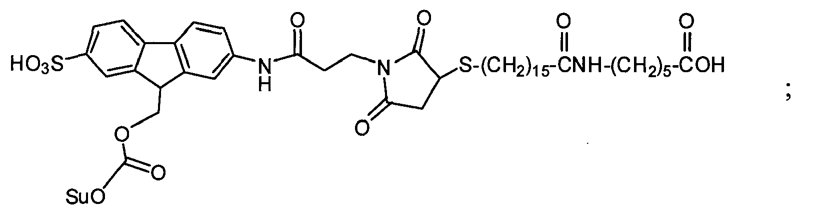
(iii) R₃ is -COOH, and R₉ is -(CH₂)₁₀-S-S-(CH₂)₁₀- group (herein identified compound 3), of the formula: 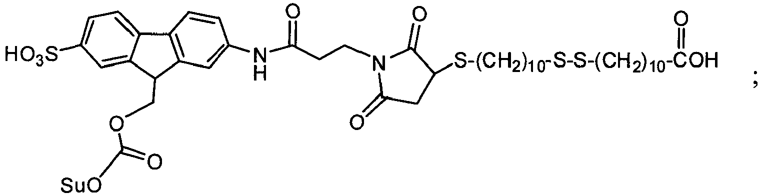 or
(iv) R₃ is -SO₃H, and R₉ is -(CH₂)₁₅-CO-NH-(CH₂)₂- group (herein identified compound **4**), of the formula:

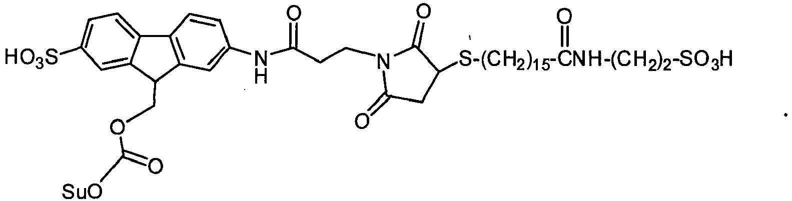

6. The compound of claim 4, wherein R₂ is R₉, and:
(i) R₃ is -COOH, and R₉ is -(CH₂)₁₅- (herein identified compound 5), of the formula: 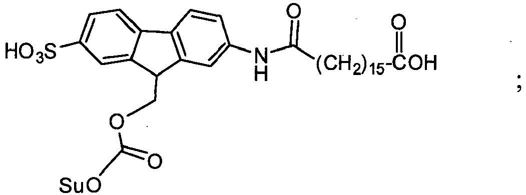
(ii) R₃ is -COOH, and R₉ is -(CH₂)₁₅-CO-NH-(CH₂)₅- group (herein identified compound **6**), of the formula: 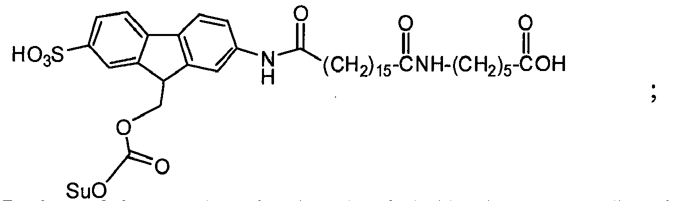
(iii) R₃ is -COOH, and R₉ is -(CH₂)₁₀-S-S-(CH₂)₁₀- group (herein identified compound 7), of the formula: 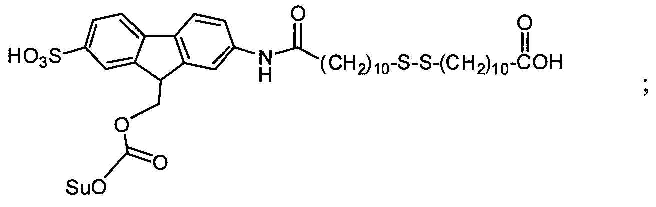 or
(iv) R₃ is -SO₃H, and R₉ is -(CH₂)₁₅-CO-NH-(CH₂)₂- (herein identified compound **8**), of the formula:

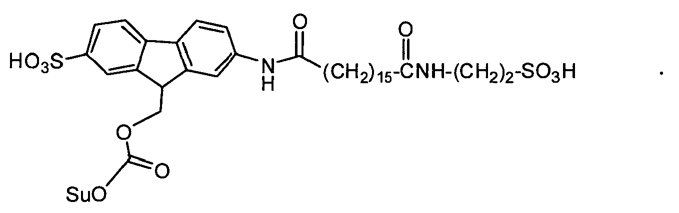

7. A conjugate of the formula II: 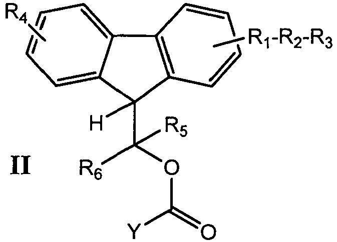 wherein
Y is a moiety of a drug containing at least one amino group, linked through said at least one amino group;
R₁ is selected from -NH-, -NH-CO-, -NH-CO-NH-, -S-, -SO₂NH-, -O-, - OCO-, -CO-NH-, -CS-NH-, -CO(CH₂)₁₋₄-, or -R₈-CO-, wherein R₈ is (C₁-C₈)alkyl optionally interrupted by a heteroatom selected from O, S or N;
R₂ is selected from 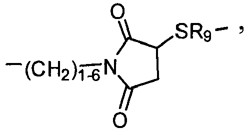 or R₉;
R₃ is an acidic group having at least one hydroxyl group such as -COOH, - SO₃H or -O-PO₃H₂;
R₄ is an electron withdrawing group such as -SO₃H, -CN, -CO-(C₁-C₈)alkyl, -CO-(C₆-C₁₀)aryl, -NO₂, -OPO₃H₂, -N(R)₃⁺, -SO₂NH₂, or halogen, wherein R is selected from (C₁-C₈)alkyl or (C₆-C₁₀)ar(C₁-C₈)alkyl;
R₅ and R₆, each independently is selected from hydrogen, -(C₁-C₈)alkyl or - (C₆-C₁₀)aryl; and
R₉ is selected from (C₁₃-C₂₀)alkylene, (C₁₃-C₂₀)alkenylene or (C₁₃-C₂₀)alkynylene, optionally interrupted by (i) a heteroatom selected from S, O or N, or more than one identical or different heteroatoms selected from S or N, and/or (ii) at least one group selected from -NH-CO-, -CO-NH-, -N(C₁-C₈alkyl)-, -N(C₆-C₁₀aryl)- or -(C₆-C₁₀)arylene-diyl-, wherein said alkenylene or alkynylene comprises one or more double or triple bond, respectively, and said one or more double or triple bond is not a terminal double or triple bond.

8. The conjugate of claim 7, wherein R₄ is -SO₃H at position 2 of the fluorene ring, and R₅ and R₆ each is hydrogen.

9. The conjugate of claim 8, wherein R₁ is -NH-CO- or -NH- at position 7 of the fluorene ring, R₂ is R₉ or 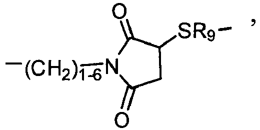 R₉ is selected from (C₁₃-C₂₀)alkylene, (C₁₃-C₂₀)alkenylene or (C₁₃-C₂₀)alkynylene, optionally interrupted by (i) a heteroatom selected from S, O or N, or more than one identical or different heteroatoms selected from S or N, (ii) and/or at least one group selected from -NH-CO-, -CO-NH-, -N(C₁-C₈alkyl)-, -N(C₆-C₁₀aryl)- or -(C₆-C₁₀)arylene-diyl-, and R₃ is -COOH or -SO₃H.

10. The conjugate of claim 9, wherein R₁ is -NH-CO-, R₂ is R₉ or 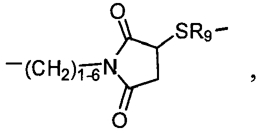 and R₉ is (C₁₃-C₂₀)alkylene, (C₁₃-C₂₀)alkenytene or (C₁₃-C₂₀)alkynylene, preferably (C₁₃-C₂₀)alkylene, optionally interrupted by (i) a heteroatom selected from S, O or N, or more than one identical or different heteroatoms selected from S or N, and/or (ii) at least one group selected from -NH-CO-, -CO-NH-, -N(C₁-C₈alkyl)-, -N(C₆-C₁₀aryl)- or -(C₆-C₁₀)arylene-diyl-.

11. The conjugate of claim 10, wherein R₂ is 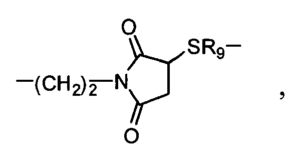 , and:
(i) R₃ is -COOH, and R₉ is -(CH₂)₁₅-, of the formula: 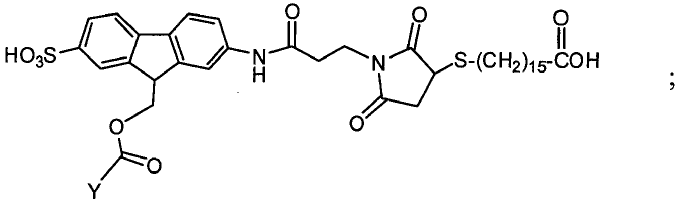
(ii) R₃ is -COOH, and R₉ is -(CH₂)₁₅-CO-NH-(CH₂)₅- group, of the formula: 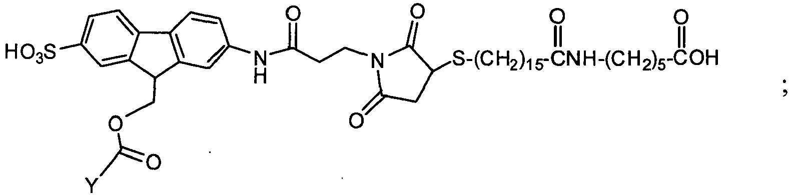
(iii) R₃ is -COOH, and R₉ is -(CH₂)₁₀-S-S-(CH₂)₁₀-group, of the formula: 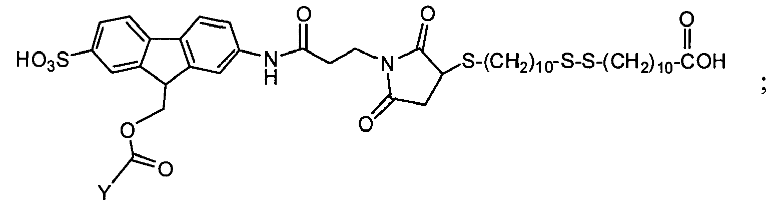 or
(iv) R₃ is -SO₃H, and R₉ is -(CH₂)₁₅-CO-NH-(CH₂)₂-, of the formula:

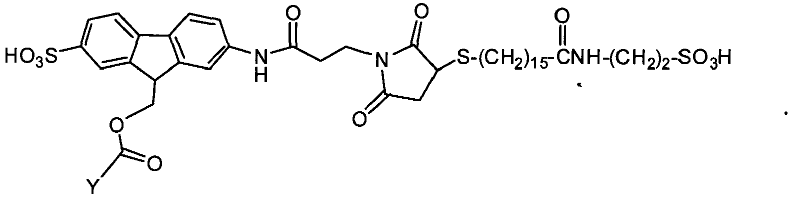

12. The conjugate of claim 10, wherein R₂ is R₉, and:
(i) R₃ is -COOH, and R₉ is -(CH₂)₁₅-, of the formula: 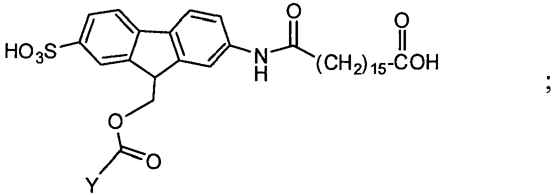
(ii) R₃ is -COOH, and R₉ is -(CH₂)₁₅-CO-NH-(CH₂)₅- group, of the formula: 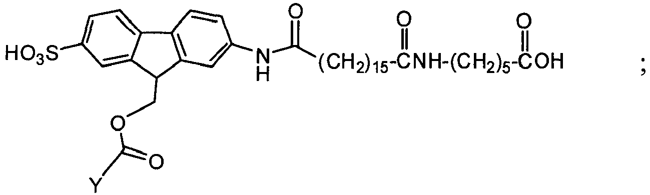
(iii) R₃ is -COOH, and R₉ is -(CH₂)₁₀-S-S-(CH₂)₁₀- group, of the formula: 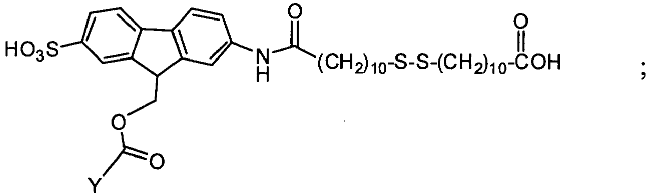 or
(iv) R₃ is -SO₃H, and R₉ is -(CH₂)₁₅-CO-NH-(CH₂)₂-, of the formula:

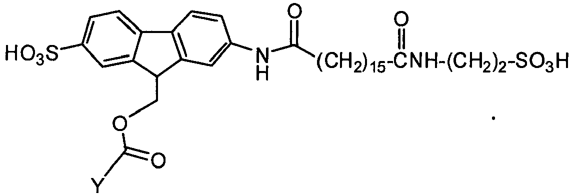

13. The conjugate of any one of claims 7 to 12, wherein:
(i) Y is an antibiotic aminoglycoside such as gentamicin or amphotericin, an antineoplastic drug such as aminolevulinic acid, or an anthracycline chemotherapeutic agent such as daunorubicin, doxorubicin, epirubicin, idarubicin, mitoxantrone and valrubicin; or
(ii) Y is a peptide or a protein drug of low or medium molecular weight such as insulin, an interferon, preferably IFN-α2, a PYY agonist, preferably the peptide PYY₃₋₃₆, an exendin, preferably exendin-3 or exendin-4, an exendin analogue or exendin agonist, atrial natriuretic peptide (ANP), human growth hormone (hGH), erythropoietin, TNF-α, calcitonin, gonadotropin releasing hormone (GnRH), a GnRH analogue, hirudin, glucagon, a coagulation factor such as Factor VIIa and Factor VIII, and a monoclonal antibody fragment, preferably anti-TNF-α monoclonal antibody fragment.

14. The conjugate of claim 11 or 12, wherein Y is insulin, exendin-4, gentamicin, Factor VIIa or Factor VIII.

15. A pharmaceutical composition comprising a conjugate according to any one of claims 7 to 14, or a pharmaceutically acceptable salt thereof, and a pharmaceutically acceptable carrier.

16. The pharmaceutical composition of claim 15, comprising a conjugate of claim 14.

17. A conjugate of formula II according to any one of claims 7 to 14, preferably according to claim 11 or 12, or a pharmaceutically acceptable salt thereof, wherein Y is insulin, for use in the treatment of diabetes mellitus or hyperglycemia.

18. A conjugate of formula II according to any one of claims 7 to 14, preferably according to claim 11 or 12, or a pharmaceutically acceptable salt thereof, wherein Y is exendin-4, for use in the treatment of insulin-dependent diabetes mellitus, non-insulin-dependent diabetes mellitus, or gestational diabetes mellitus, or for the prevention of hyperglycemia.

19. A conjugate of formula II according to any one of claims 7 to 14, preferably according to claim 11 or 12, or a pharmaceutically acceptable salt thereof, wherein Y is gentamicin, for use in the treatment of a bacterial infection, preferably a bacterial infection caused by gram-negative bacteria.

20. A conjugate of formula II according to any one of claims 7 to 14, preferably according to claim 11 or 12, or a pharmaceutically acceptable salt thereof, wherein Y is Factor VIIa or Factor VIII, for use in Factor VIIa or Factor VIII therapy, respectively.

## Patentansprüche

1. Eine Verbindung der Formel I: 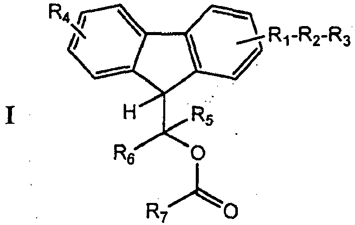 wobei
R₁ ausgewählt ist aus -NH-, -NH-CO-, -NH-CO-NH-, -S-, -SO₂NH-, -O-, -OCO-, -CO-NH-, -CS-NH-, -CO(CH₂)₁₋₄- oder -R₈-CO-, wobei R₈ für (C₁-C₈)-Alkyl steht, welches gegebenenfalls durch ein Heteroatom ausgewählt aus O, S oder N unterbrochen ist;
R₂ ausgewählt ist aus 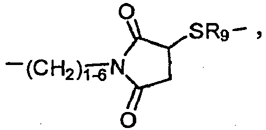 oder R₉;
R₃ eine saure Gruppe mit mindestens einer Hydroxylgruppe wie -COOH, -SO₃H oder -O-PO₃H₂ darstellt;
R₄ eine elektronenziehende Gruppe wie -SO₃H, -CN, -CO-(C₁-C₈)-Alkyl, -CO-(C₆-C₁₀)-Aryl, -NO₂, -OPO₃H₂, -N(R)₃⁺, -SO₂NH₂ oder Halogen darstellt, wobei R ausgewählt ist aus (C₁-C₈)-Alkyl oder (C₆-C₁₀)ar(C₁C₈)-Alkyl;
R₅ und R₆ jeweils unabhängig ausgewählt sind aus Wasserstoff, -(C₁-C₈)-Alkyl oder -(C₆-C₁₀)-Aryl;
R₇ eine Abgangsgruppe ist wie -O-(CH₂)₂-CN, -Cl, 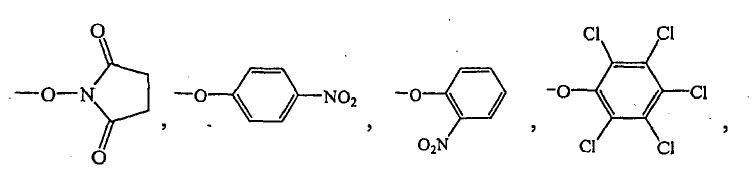 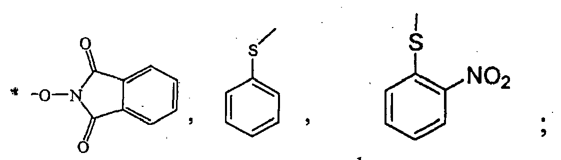 und
R₉ ausgewählt ist aus (C₁₃-C₂₀)-Alkylen, (C₁₃-C₂₀)-Alkenylen oder (C₁₃-C₂₀)-Alkinylen, gegebenenfalls unterbrochen durch (i) ein Heteroatom ausgewählt aus S, O oder N, oder mehr als ein identisches oder unterschiedliches Heteroatom ausgewählt aus S oder N, und/oder (ii) mindestens eine Gruppe ausgewählt aus -NH-CO-, -CO-NH-, -N(C₁-C₈-Alkyl)-, -N(C₆-C₁₀-Aryl)-, oder -(C₆-C₁₀)-Arylen-diyl-, wobei das Alkenylen oder Alkinylen eine oder mehrere Doppel- oder Dreifachbindungen umfasst, und die eine oder mehrere Doppel- oder Dreifachbiridungen keine endständige Doppel- oder Dreifachbindung sind.

2. Die Verbindung gemäß Anspruch 1, wobei R₄ für -SO₃H an Position 2 des Fluorenrings steht, R₅ und R₆ jeweils Wasserstoff darstellen und R₇ für N-Hydroxysuccinimid (-OSu) steht.

3. Die Verbindung gemäß Anspruch 2, wobei R₁ für -NH-CO- oder -NH- an Position 7 des Fluorenrings steht, R₂ für R₉ oder 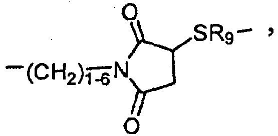 steht,
R₉ ausgewählt ist aus (C₁₃-C₂₀)-Alkylen, (C₁₃-C₂₀)-Alkenylen oder (C₁₃-C₂₀)-Alkinylen, gegebenenfalls unterbrochen durch (i) ein Heteroatom ausgewählt aus S, O oder N, oder mehr als ein identisches oder unterschiedliches Heteroatom ausgewählt aus S oder N, und/oder (ii) mindestens eine Gruppe ausgewählt aus -NH-CO-, -CO-NH-, -N(C₁-C₈-Alkyl)-, -N(C₆-C₁₀-Aryl)- oder -(C₆-C₁₀)-Arylen-diyl-, und R₃ für -COOH oder -SO₃H steht.

4. Die Verbindung gemäß Anspruch 3, wobei R₁ für -NH-CO- steht, R₂ für R₉ oder 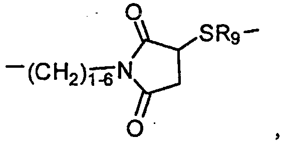 steht,
und R₉ für (C₁₃-C₂₀)-Alkylen, (C₁₃-C₂₀)-Alkenylen oder (C₁₃-C₂₀)-Alkinylen steht, vorzugsweise (C₁₃-C₂₀)-Alkylen, gegebenenfalls unterbrochen durch (i) ein Heteroatom ausgewählt aus S, O oder N, oder mehr als ein identisches oder unterschiedliches Heteroatom ausgewählt aus S oder N, und/oder (ii) mindestens eine Gruppe ausgewählt aus -NH-CO-, -CO-NH-, -N(C₁-C₈-Alkyl)-, -N(C₆-C₁₀-Aryl)- oder -(C₆-C₁₀)-Arylen-diyl-.

5. Die Verbindung gemäß Anspruch 4, wobei R₂ für 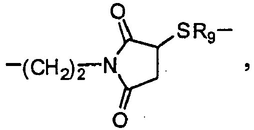 steht; und:
(i) R₃ für -COOH steht und R₉ eine Gruppe -(CH₂)₁₅- ist (hier als Verbindung **1** bezeichnet), der Formel 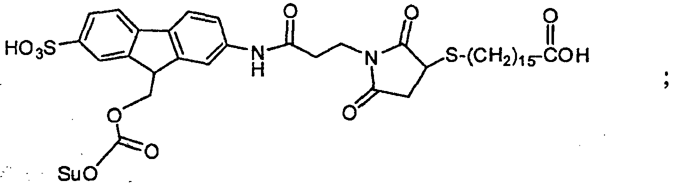
(ii) R₃ für -COOH steht und R₉ eine Gruppe -(CH₂)₁₅-CO-NH-(CH₂)₅- ist (hier als Verbindung 2 bezeichnet), der Formel: 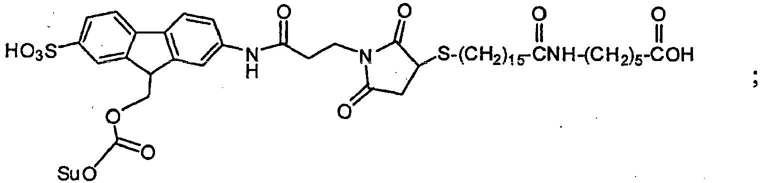
(iii) R₃ für -COOH steht und R₉ eine Gruppe -(CH₂)₁₀-S-S-(CH₂)₁₀- ist (hier als Verbindung **3** bezeichnet), der Formel: 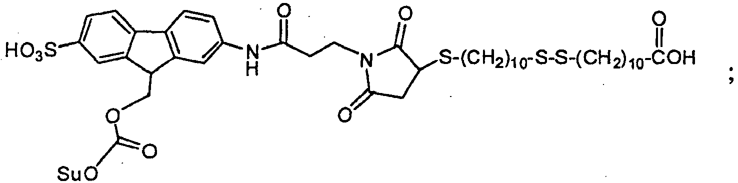 oder
(iv) R₃ für -SO₃H steht und R₉ eine Gruppe -(CH₂)₁₅-CO-NH-(CH₂)₂- ist (hier als Verbindung **4** bezeichnet), der Formel:

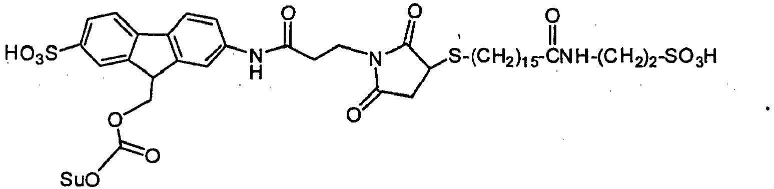

6. Die Verbindung gemäß Anspruch 4, wobei R₂ für R₉ steht und:
(i) R₃ für -COOH steht und R₉ für -(CH₂)₁₅- steht (hier als Verbindung **5** bezeichnet), der Formel: 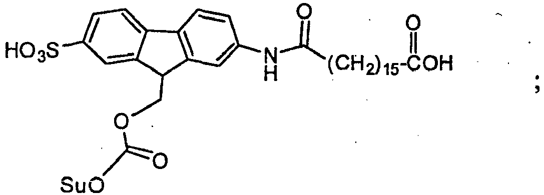
(ii) R₃ für -COOH steht und R₉ eine Gruppe -(CH₂)₁₅-CO-NH-(CH₂)₅- ist (hier als Verbindung **6** bezeichnet), der Formel: 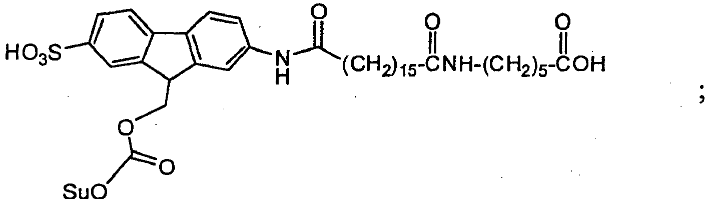
(iii) R₃ für -COOH steht und R₉ eine Gruppe -(CH₂)₁₀-S-S-(CH₂)₁₀- ist (hier als Verbindung **7** bezeichnet), der Formel: 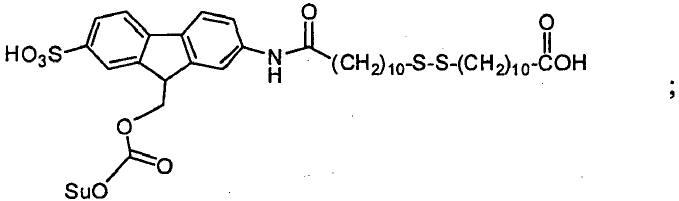 oder
(iv) R₃ für -SO₃H steht und R₉ eine Gruppe -(CH₂)₁₅-CO-NH-(CH₂)₂- ist (hier als Verbindung **8** bezeichnet), der Formel:

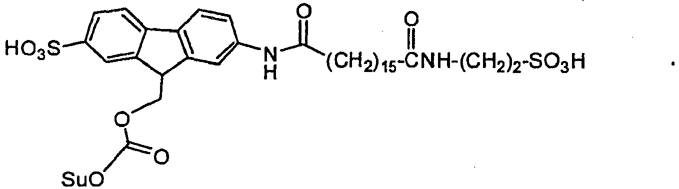

7. Ein Konjugat der Formel II: 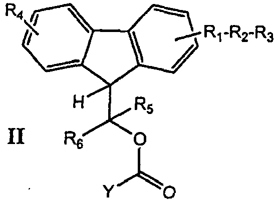 wobei
Y eine mindestens eine Aminogruppe enthaltende Einheit eines Arzneistoffs, welche durch diese mindestens eine Aminogruppe gebunden ist, ist;
R₁ ausgewählt ist aus -NH-, -NH-CO-, -NH-CO-NH-, -S-, -SO₂NH-, -O-, -OCO-, -CO-NH-, -CS-NH-, -CO(CH₂)₁₋₄- oder -R₈-CO-, wobei R₈ für (C₁-C₈)-Alkyl steht, welches gegebenenfalls durch ein Heteroatom ausgewählt aus O, S oder N unterbrochen ist;
R₂ ausgewählt ist aus 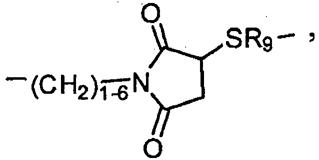 oder R₉;
R₃ eine saure Gruppe mit mindestens einer Hydroxylgruppe wie -COOH, -SO₃H oder -O-PO₃H₂ darstellt;
R₄ eine elektronenziehende Gruppe wie -SO₃H, -CN, -CO-(C₁-C₈)-Alkyl, -CO-(C₆-C₁₀)-Aryl, -NO₂, -OPO₃H₂, -N(R)₃⁺, -SO₂NH₂ oder Halogen ist, wobei R ausgewählt ist aus (C₁-C₈)-Alkyl oder (C₆-C₁₀)ar(C₁-C₈)-Alkyl;
R₅ und R₆ jeweils unabhängig ausgewählt sind aus Wasserstoff, -(C₁-C₈)-Alkyl oder -(C₆-C₁₀)-Aryl; und
R₉ ausgewählt ist aus (C₁₃-C₂₀)-Alkylen, (C₁₃-C₂₀)-Alkenylen oder (C₁₃-C₂₀)-Alkinylen, gegebenenfalls unterbrochen durch (i) ein Heteroatom ausgewählt aus S, O oder N, oder mehr als ein identisches oder unterschiedliches Heteroatom ausgewählt aus S oder N, und/oder (ii) mindestens eine Gruppe ausgewählt aus -NH-CO-, -CO-NH-, -N(C₁C₈-Alkyl)-, -N(C₆-C₁₀-Aryl)- oder -(C₆-C₁₀)-Arylen-diyl-, wobei das Alkenylen oder Alkinylen eine oder mehrere Doppel- oder Dreifachbindungen umfasst und die eine oder mehrere Doppel- oder Dreifachbindungen keine endständige Doppel- oder Dreifachbindung ist.

8. Das Konjugat gemäß Anspruch 7, wobei R₄ für -SO₃H an Position 2 des Fluorenrings steht und R₅ und R₆ jeweils Wasserstoff darstellen.

9. Das Konjugat gemäß Anspruch 8, wobei R₁ für -NH-CO- oder -NH- an Position 7 des Fluorenrings steht, R₂ gleich R₉ oder 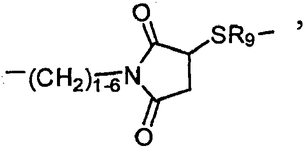 ist,
R₉ ausgewählt ist aus (C₁₃-C₂₀)-Alkylen, (C₁₃-C₂₀)-Alkenylen oder (C₁₃-C₂₀)-Alkinylen, gegebenenfalls unterbrochen durch (i) ein Heteroatom ausgewählt aus S, O oder N, oder mehr als ein identisches oder unterschiedliches Heteroatom ausgewählt aus S oder N, und/oder (ii) mindestens eine Gruppe ausgewählt aus -NH-CO-, -CO-NH-, -N(C₁-C₈-Alkyl)-, -N(C₆-C₁₀-Aryl)- oder -(C₆-C₁₀)-Arylen-diyl-, und R₃ für -COOH oder -SO₃H steht.

10. Das Konjugat gemäß Anspruch 9, wobei R₁ für -NH-CO- steht, R₂ gleich R₉ oder 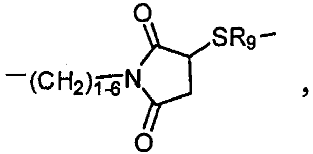 ist,
und R₉ für (C₁₃-C₂₀)-Alkylen, (C₁₃-C₂₀)-Alkenylen oder (C₁₃-C₂₀)-Alkinylen steht, vorzugsweise (C₁₃-C₂₀)-Alkylen, gegebenenfalls unterbrochen durch (i) ein Heteroatom ausgewählt aus S, O oder N, oder mehr als ein identisches oder unterschiedliches Heteroatom ausgewählt aus S oder N, und/oder (ii) mindestens eine Gruppe ausgewählt aus -NH-CO-, -CO-NH-, -N(C₁-C₈-Alkyl)-, -N(C₆-C₁₀-Aryl)- oder -(C₆-C₁₀)-Arylen-diyl-.

11. Das Konjugat gemäß Anspruch 10, wobei R₂ für 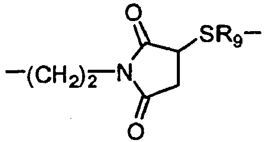 steht; und
(i) R₃ für -COOH steht und R₉ für -(CH₂)₁₅- steht, der Formel: 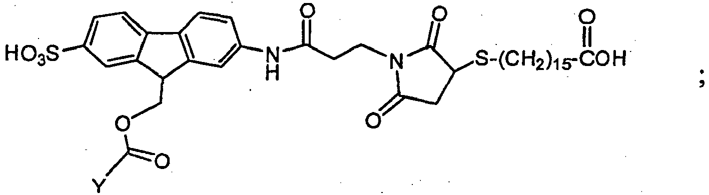
(ii) R₃ für -COOH steht und R₉ die Gruppe -(CH₂)₁₅-CO-NH-(CH₂)₅- ist, der Formel: 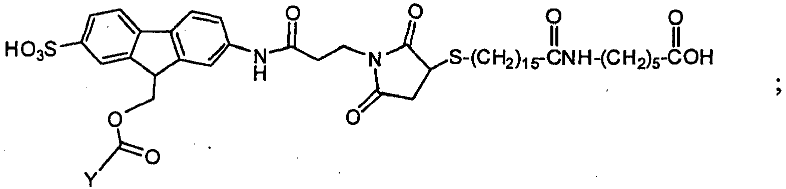
(iii) R₃ für -COOH steht und R₉ die Gruppe -(CH₂)₁₀-S-S-(CH₂)₁₀- ist, der Formel: 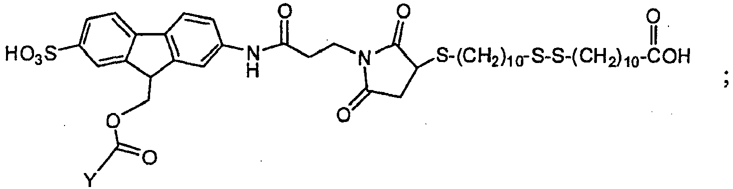 oder
(iv) R₃ für -SO₃H steht und R₉ für -(CH₂)₁₅-CO-NH-(CH₂)₂- steht, der Formel:

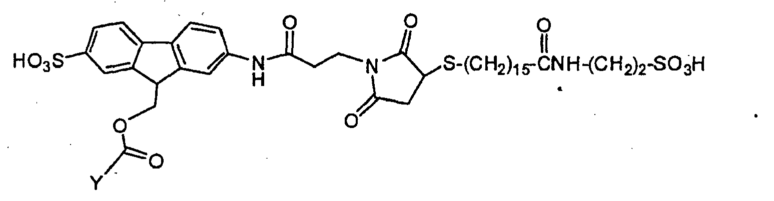

12. Das Konjugat gemäß Anspruch 10, wobei R₂ für R₉ steht und:
(i) R₃ für -COOH steht und R₉ für -(CH₂)₁₅- steht, der Formel: 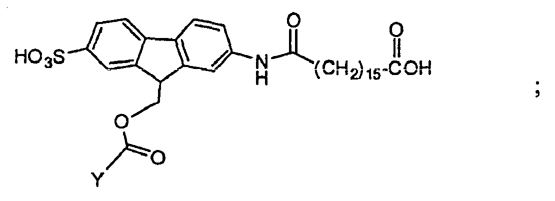
(ii) R₃ für -COOH steht und R₉ die Gruppe -(CH₂)₁₅-CO-NH-(CH₂)₅- ist, der Formel: 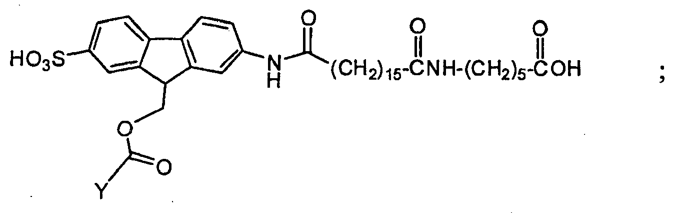 steht;
(iii) R₃ für -COOH steht und R₉ die Gruppe -(CH₂)₁₀-S-S-(CH₂)₁₀- ist, der Formel: 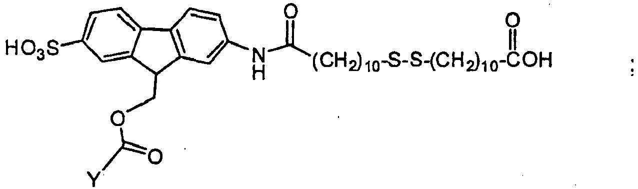 oder
(iv) R₃ für -SO₃H steht und R₉ für -(CH₂)₁₅-CO-NH-(CH₂)₂- steht, der Formel:

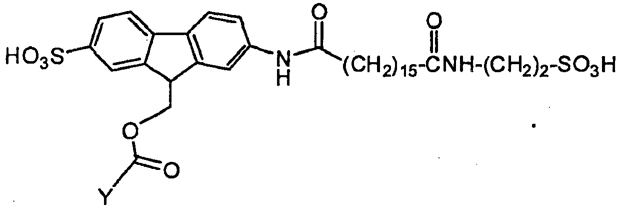

13. Das Konjugat gemäß einem der Ansprüche 7 bis 12, wobei:
(i) Y ein antibiotisches Aminoglycosid wie Gentamicin oder Amphotericin, ein antineoplastisches Medikament wie Aminolävulinsäure, oder ein Anthracyclin-Chemotherapeutikum wie Daunorubicin, Doxorubicin, Epirubicin, Idarubicin, Mitoxantron und Valrubicin ist; oder
(ii) Y ein Peptid- oder Proteinarzneistoff von geringem oder mittlerem Molekulargewicht wie Insulin, ein Interferon, vorzugweise IFN-alpha2, ein PYY-Agonist, vorzugsweise das Peptid PYY₃₋₃₆, ein Exendin, vorzugsweise Exendin-3 oder Exendin-4, ein Exendin-Analog oder Exendin-Agonist, atriales natriuretisches Peptid (ANP), humanes Wachstumshormon (hGH), Erythropoietin, TNF-alpha, Calcitonin, Gonadotropin freisetzendes Hormon (GnRH), ein GnRH-Analog, Hirudin, Glucagon, ein Koagulationsfaktor wie Faktor VIIa und Faktor VIII, und ein monoklonales Antikörperfragment, vorzugsweise Anti-TNF-alpha monoklonales Antikörperfragment, ist

14. Das Konjugat gemäß Anspruch 11 oder 12, wobei Y für Insulin, Exendin-4, Gentamicin, Faktor VIIa oder Faktor VIII steht.

15. Ein Arzneimittel, umfassend ein Konjugat gemäß einem der Ansprüche 7 bis 14, oder ein pharmazeutisch verträgliches Salz davon und einen pharmazeutisch verträglichen Träger.

16. Das Arzneimittel gemäß Anspruch 15, umfassend ein Konjugat gemäß Anspruch 14.

17. Ein Konjugat der Formel II gemäß einem der Ansprüche 7 bis 14, vorzugsweise gemäß Anspruch 11 oder 12, oder ein pharmazeutisch verträgliches Salz davon, wobei Y Insulin ist, zur Verwendung bei der Behandlung von Diabetes mellitus oder Hyperglykämie.

18. Ein Konjugat der Formel II gemäß einem der Ansprüche 7 bis 14, vorzugsweise gemäß Anspruch 11 oder 12, oder ein pharmazeutisch verträgliches Salz davon, wobei Y Exendin-4 ist, zur Verwendung bei der Behandlung von Insulin-abhängigem Diabetes mellitus, nicht-Insulin-abhängigem Diabetes mellitus oder Schwangerschaftsdiabetes oder zur Vorbeugung von Hyperglykämie.

19. Ein Konjugat der Formel II gemäß einem der Ansprüche 7 bis 14, vorzugsweise gemäß Anspruch 11 oder 12, oder ein pharmazeutisch verträgliches Salz davon, wobei Y Gentamicin ist, zur Verwendung bei der Behandlung einer bakteriellen Infektion, vorzugsweise einer durch gram-negative Bakterien verursachten bakteriellen Infektion.

20. Ein Konjugat der Formel II gemäß einem der Ansprüche 7 bis 14, vorzugsweise gemäß Anspruch 11 oder 12 oder ein pharmazeutisch verträgliches Salz davon, wobei Y Faktor VIIa oder Faktor VIII ist, zur Verwendung bei der Faktor-VIIa oder Faktor-VIII-Therapie.

## Revendications

1. Composé de formule I: 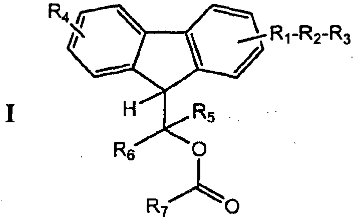 dans laquelle
R₁ est choisi parmi les groupes -NH-, -NH-CO-, NH-CO-NH-, -S-, -SO₂NH-, -O-, -OCO-, -CO-NH-, -CS-NH-, -CO(CH₂)₁₋₄-, ou -R₈-CO-, où R₈ représente un groupe alkyle (en C₁ à C₈) éventuellement interrompu par un hétéroatome choisi parmi O, S ou N ;
R₂ est choisi parmi 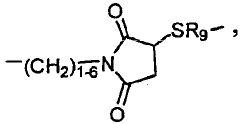 ou R₉;
R₃ représente un groupe acide comportant au moins un groupe hydroxyle tel que -COOH, -SO₃H ou -O-PO₃H₂ ;
R₄ représente un groupe accepteur d'électron tel que -SO₃H, -CN, -CO-alkyle (en C₁ à C₈), -CO-aryle (en C₆ à C₁₀), -NO₂, -OPO₃H₂, -N(R)₃⁺, -SO₂NH₂, ou halogéno, où R est choisi parmi les groupes alkyle (en C₁ à C₈) ou aryl (en C₆ à C₁₀)-alkyle (en C₁ à C₈) ;
R₅ et R₆, chacun indépendamment, sont choisis parmi l'atome d'hydrogène, les groupes -alkyle (en C₁ à C₈) ou aryle (en C₆ à C₁₀) ;
R₇ représente un groupe partant tel que -O-(CH₂)₂-CN, -Cl, 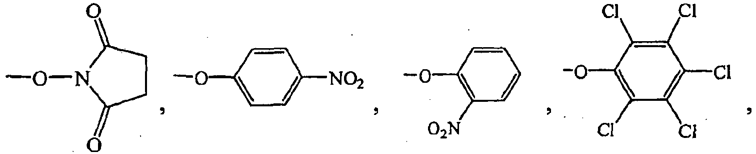 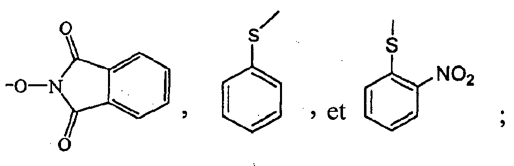 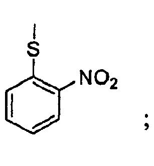 et R₉ est choisi parmi les groupes alkylène (en C₁₃ à C₂₀), alcénylène (en C₁₃ à C₂₀) ou alcynylène (en C₁₃ à C₂₀), éventuellement interrompu par (i) un hétéroatome choisi parmi S, O ou N, ou plus d'un hétéroatome identique ou différent choisi parmi S ou N, et/ou (ii) au moins un groupe choisi parmi les groupes -NH-CO-, -CO-NH-, -N-(alkyl en C₁ à C₈)-, -N-(aryl en C₆ à C₁₀)-, ou -arylène (en C₆ à C₁₀)-diyl-, où ledit groupe alcénylène ou alcynylène comprend une ou plusieurs doubles ou triples liaisons, respectivement, et lesdites une ou plusieurs doubles ou triples liaisons ne sont pas une double ou une triple liaison terminale.

2. Composé selon la revendication 1, dans lequel R₄ représente un groupe -SO₃H au niveau de la position 2 du cycle fluorène, R₅ et R₆ représentent chacun un atome d'hydrogène, et R₇ représente un groupe N-hydroxysuccinimide (-OSu).

3. Composé selon la revendication 2, dans lequel R₁ représente un groupe NH-CO- ou -NH- au niveau de la position 7 du cycle fluorène, R₂ représente R₉ ou 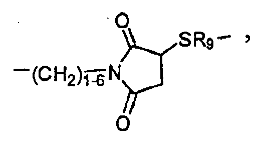 R₉ est choisi parmi les groupes alkylène (en C₁₃ à C₂₀), alcénylène (en C₁₃ à C₂₀) ou alcynylène (en C₁₃ à C₂₀), éventuellement interrompu par (i) un hétéroatome choisi parmi S, O ou N, ou plus d'un hétéroatome identique ou différent choisi parmi S ou N, et/ou (ii) au moins un groupe choisi parmi les groupes -NH-CO-, -CO-NH-, -N-(alkyl en C₁ à C₈)-, N-(aryl en C₆ à C₁₀)-, ou -arylène (en C₆ à C₁₀)-diyl-, et R₃ représente un groupe -COOH ou -SO₃H.

4. Composé selon la revendication 3, dans lequel R₁ représente un groupe -NH-CO- R₂ représente R₉ ou 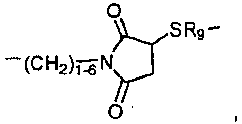 et R₉ est choisi parmi les groupes alkylène (en C₁₃ à C₂₀), alcénylène (en C₁₃ à C₂₀) ou alcynylène (en C₁₃ à C₂₀), de préférence alkylène (en C₁₃ à C₂₀), éventuellement interrompu par (i) un hétéroatome choisi parmi S, O ou N, ou plus d'un hétéroatome identique ou différent choisi parmi S ou N, et/ou (ii) au moins un groupe choisi parmi les groupes -NH-CO-, -CO-NH-, -N-(alkyl en C₁ à C₈)-, N-(aryl en C₆ à C₁₀)-, ou -arylène (en C₆ à C₁₀)-diyl-.

5. Composé selon la revendication 4, dans lequel R₂ représente et : 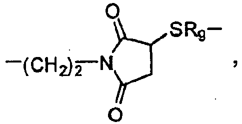
(i) R₃ représente un groupe -COOH, et R₉ représente un groupe -(CH₂)₁₅-(identifié ici comme le composé 1), de formule : 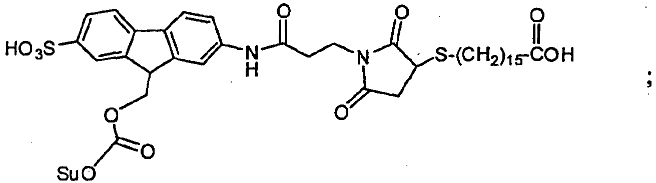
(ii) R₃ représente un groupe -COOH, et R₉ représente un groupe -(CH₂)₁₅-CO-NH-(CH₂)₅- (identifié ici comme le composé 2), de formule : 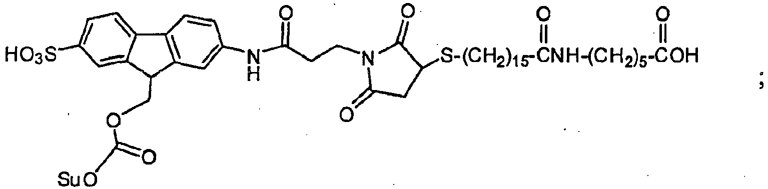
(iii) R₃ représente un groupe -COOH, et R₉ représente un groupe -(CH₂)₁₀-S-S-(CH₂)₁₀- (identifié ici comme le composé 3), de formule : 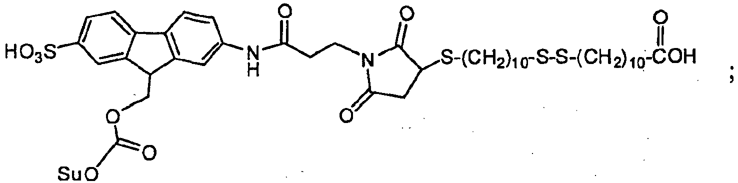 ou
(iv) R₃ représente un groupe -SO₃H, et R₉ représente un groupe -(CH₂)₁₅-CO-NH-(CH₂)₂- (identifié ici comme le composé 4), de formule :

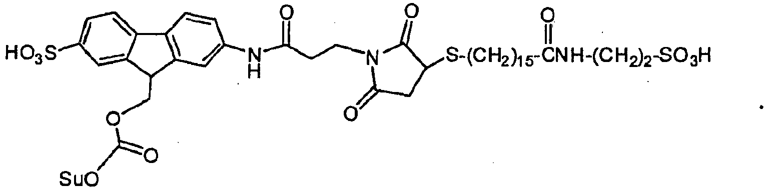

6. Composé selon la revendication 4, dans lequel R₂ représente R₉, et :
(i) R₃ représente un groupe -COOH, et R₉ représente un groupe -(CH₂)₁₅- (identifié ici comme le composé 5), de formule : 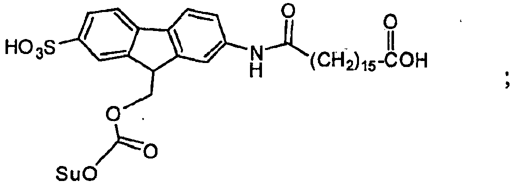
(ii) R₃ représente un groupe -COOH, et R₉ représente un groupe -(CH₂)₁₅-CO-NH-(CH₂)₅- (identifié ici comme le composé 6), de formule : 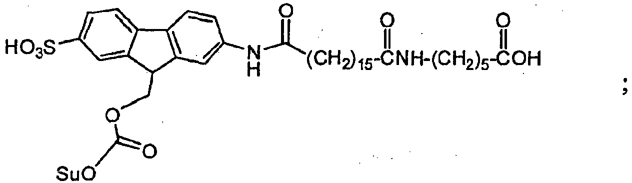
(iii) R₃ représente un groupe -COOH, et R₉ représente un groupe -(CH₂)₁₀-S-S-(CH₂)₁₀- (identifié ici comme le composé 7), de formule : 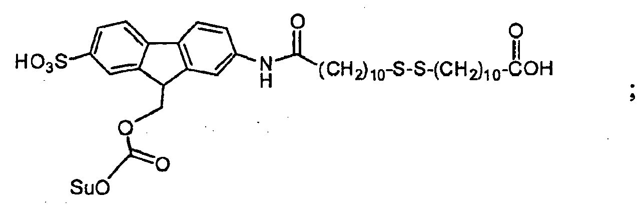 ou
(iv) R₃ représente un groupe -SO₃H, et R₉ représente un groupe -(CH₂)₁₅-CO-NH-(CH₂)₂- (identifié ici comme le composé 8), de formule :

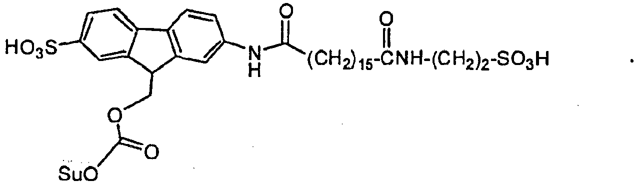

7. Conjugué de formule II : 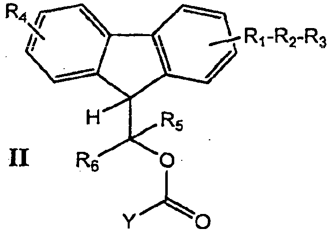 dans laquelle
Y représente un radical d'un médicament contenant au moins un groupe amino, lié par l'intermédiaire dudit au moins un groupe amino ;
R₁ est choisi parmi les groupes -NH-, -NH-CO-, -NH-CO-NH-, -S-, -SO₂NH-, -O-, -OCO-, -CO-NH-, -CS-NH-, -CO(CH₂)₁₋₄-, ou -R₈-CO-, où R₈ représente un groupe alkyle (en C₁ à C₈) éventuellement interrompu par un hétéroatome choisi parmi O, S ou N ;
R₂ est choisi parmi 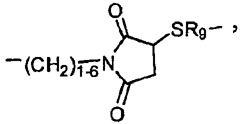 ou R₉;
R₃ représente un groupe acide comportant au moins un groupe hydroxyle tel que -COOH, -SO₃H ou -O-PO₃H₂ ;
R₄ représente un groupe attracteur d'électron tel que -SO₃H, -CN, -CO-alkyle (en C₁ à C₈), -CO-aryle (en C₆ à C₁₀), -NO₂, -OPO₃H₂, -N(R)₃⁺, -SO₂NH₂, ou halogéno, où R est choisi parmi les groupes alkyle (en C₁ à C₈) ou aryl (en C₆ à C₁₀)-alkyle (en C₁ à C₈) ;
R₅ et R₆, chacun indépendamment, sont choisis parmi l'atome d'hydrogène, les groupes -alkyle (en C₁ à C₈) ou -aryle (en C₆ à C₁₀) ; et
R₉ est choisi parmi les groupes alkylène (en C₁₃ à C₂₀), alcénylène (en C₁₃ à C₂₀) ou alcynylène (en C₁₃ à C₂₀), éventuellement interrompu par (i) un hétéroatome choisi parmi S, O ou N, ou plus d'un hétéroatome identique ou différent choisi parmi S ou N, et/ou (ii) au moins un groupe choisi parmi les groupes -NH-CO-, -CO-NH-, -N-(alkyl en C₁ à C₈)-, -N-(aryl en C₆ à C₁₀)-, ou -arylène (en C₆ à C₁₀)-diyl-, où ledit groupe alcénylène ou alcynylène comprend une ou plusieurs doubles ou triples liaisons, respectivement, et lesdites une ou plusieurs doubles ou triples liaisons ne sont pas une double ou une triple liaison terminale.

8. Conjugué selon la revendication 7, dans lequel R₄ représente un groupe -SO₃H au niveau de la position 2 du cycle fluorène, et R₅ et R₆ représentent chacun un atome d'hydrogène.

9. Conjugué selon la revendication 8, dans lequel R₁ représente un groupe -NH-CO- ou -NH- au niveau de la position 7 du cycle fluorène, R₂ représente R₉ ou 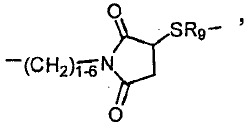 R₉ est choisi parmi les groupes alkylène (en C₁₃ à C₂₀), alcénylène (en C₁₃ à C₂₀) ou alcynylène (en C₁₃ à C₂₀), éventuellement interrompu par (i) un hétéroatome choisi parmi S, O ou N, ou plus d'un hétéroatome identique ou différent choisi parmi S ou N, et/ou (ii) au moins un groupe choisi parmi les groupes -NH-CO-, -CO-NH-, -N-(alkyl en C₁ à C₈)-, -N-(aryl en C₆ à C₁₀)-, ou -arylène (en C₆ à C₁₀)-diyl-, et R₃ représente un groupe -COOH ou -SO₃H.

10. Conjugué selon la revendication 9, dans lequel R₁ représente un groupe -NH-CO-, R₂ représente R₉ ou 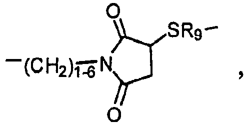 et R₉ est choisi parmi les groupes alkylène (en C₁₃ à C₂₀), alcénylène (en C₁₃ à C₂₀) ou alcynylène (en C₁₃ à C₂₀), de préférence alkylène (en C₁₃ à C₂₀), éventuellement interrompu par (i) un hétéroatome choisi parmi S, O ou N, ou plus d'un hétéroatome identique ou différent choisi parmi S ou N, et/ou (ii) au moins un groupe choisi parmi les groupes -NH-CO-, -CO-NH-, N-(alkyl en C₁ à C₈)-, -N-(aryl en C₆ à C₁₀)-, ou -arylène (en C₆ à C₁₀)-diyl-.

11. Conjugué selon la revendication 10, dans lequel R₂ représente 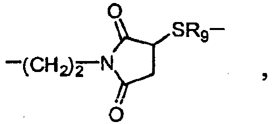 et :
(i) R₃ représente un groupe -COOH, et R₉ représente un groupe -(CH₂)₁₅-, de formule : 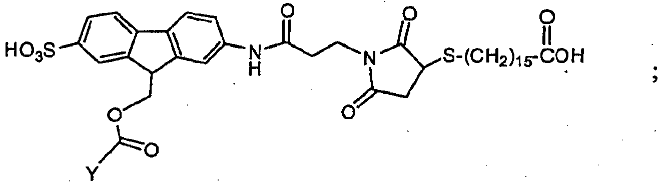
(ii) R₃ représente un groupe -COOH, et R₉ représente un groupe -(CH₂)₁₅-CO-NH-(CH₂)₅-, de formule : 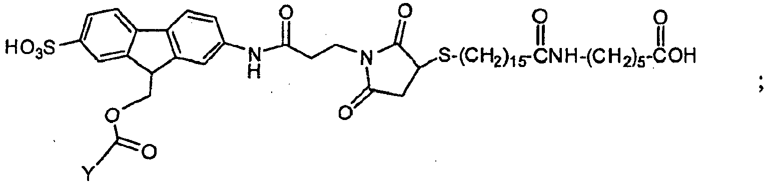
(iii) R₃ représente un groupe -COOH, et R₉ représente un groupe -(CH₂)₁₀-S-S-(CH₂)₁₀-, de formule : 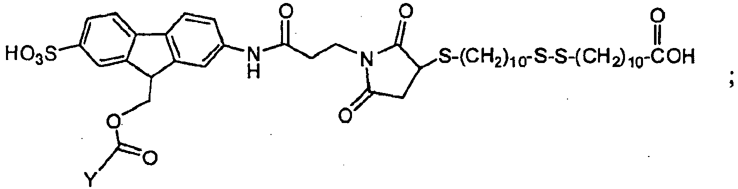 ou
(iv) R₃ représente un groupe -SO₃H, et R₉ représente un groupe -(CH₂)₁₅-CO-NH-(CH₂)₂-, de formule :

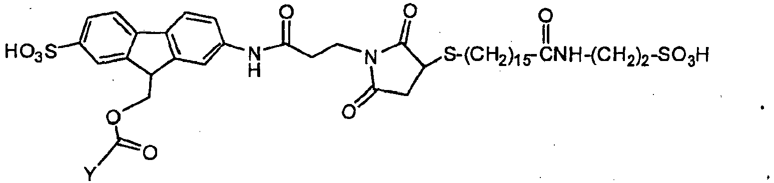

12. Conjugué selon la revendication 10, dans lequel R₂ représente R₉, et :
(i) R₃ représente un groupe -COOH, et R₉ représente un groupe -(CH₂)₁₂-, de formule : 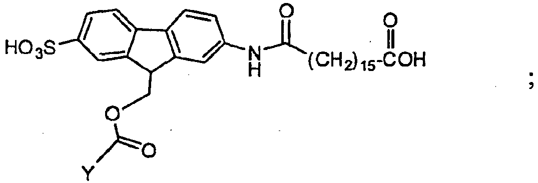
(ii) R₃ représente un groupe -COOH, et R₉ représente un groupe -(CH₂)₁₅-CO-NH-(CH₂)₅-, de formule : 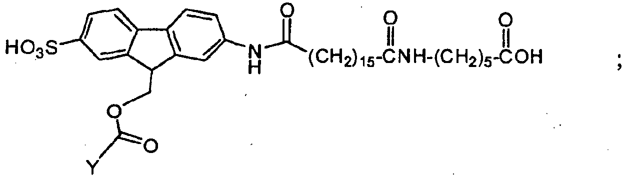
(iii) R₃ représente un groupe -COOH, et R₉ représente un groupe -(CH₂)₁₀-S-S-(CH₂)₁₀-, de formule : 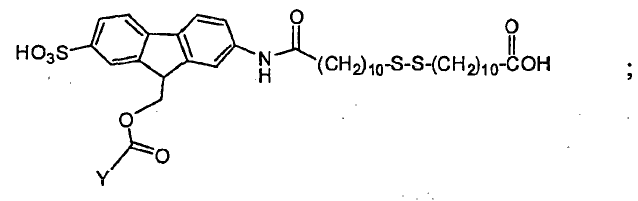 ou
(iv) R₃ représente un groupe -SO₃H, et R₉ représente un groupe -(CH₂)₁₅-CO-NH-CH₂)₂-, de formule :

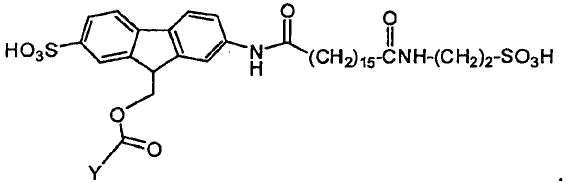

13. Conjugué selon l'une quelconque des revendications 7 à 12, dans lequel :
(i) Y représente un antibiotique aminoglycoside tel que la gentamycine ou l'amphotéricine, un médicament antinéoplasique tel que l'acide aminolévulinique, ou un agent chimiothérapeutique à base d'anthracycline tel que la daunorubicine, la doxorubicine, l'épirubicine, l'idarubicine, la mitoxantrone et la valrubicine ; ou
(ii) Y représente un médicament à base d'un peptide ou d'une protéine de poids moléculaire bas ou moyen tel que l'insuline, un interféron, de préférence l'IFN-α2, un agoniste du PYY, de préférence du peptide PYY₃₋₃₆, une exendine, de préférence l'exendine-3 ou l'exendine-4, un analogue d'exendine ou un agoniste d'exendine, le peptide natriurétique auriculaire (ANP), l'hormone de croissance humaine (hGH), l'érythropoïétine, le TNF-α, la calcitonine, l'hormone de libération des gonadotrophines (GnRH), un analogue de la GnRH, l'hirudine, le glucagon, un facteur de coagulation comme le facteur VIIa et le facteur VIII, et un fragment d'anticorps monoclonal, de préférence un fragment d'anticorps monoclonal anti-TNF-α.

14. Conjugué selon la revendication 11 ou 12, dans lequel Y représente l'insuline, l'exendine-4, la gentamycine, le facteur VIIa ou le facteur VIII.

15. Composition pharmaceutique comprenant un conjugué selon l'une quelconque des revendications 7 à 14, ou l'un de ses sels pharmaceutiquement acceptables, et un support pharmaceutiquement acceptable.

16. Composition pharmaceutique selon la revendication 15, comprenant un conjugué selon la revendication 14.

17. Conjugué de formule II selon l'une quelconque des revendications 7 à 14, de préférence selon la revendication 11 ou 12, ou l'un de ses sels pharmaceutiquement acceptables, dans lequel Y représente l'insuline, pour une utilisation dans le traitement du diabète ou de l'hyperglycémie.

18. Conjugué de formule II selon l'une quelconque des revendications 7 à 14, de préférence selon la revendication 11 ou 12, ou l'un de ses sels pharmaceutiquement acceptables, dans lequel Y représente l'exendine-4, pour une utilisation dans le traitement du diabète insulinodépendant, du diabète non insulinodépendant, ou du diabète gestationnel, ou pour la prévention de l'hyperglycémie.

19. Conjugué de formule II selon l'une quelconque des revendications 7 à 14, de préférence selon la revendication 11 ou 12, ou l'un de ses sels pharmaceutiquement acceptables, dans lequel Y représente la gentamycine, pour une utilisation dans le traitement d'une infection bactérienne, de préférence une infection bactérienne provoquée par des bactéries Gram négatif.

20. Conjugué de formule II selon l'une quelconque des revendications 7 à 14, de préférence selon la revendication 11 ou 12, ou l'un de ses sels pharmaceutiquement acceptables, dans lequel Y représente le facteur VIIa ou le facteur VIII, pour une utilisation dans une thérapie par le facteur VIIa ou le facteur VIII, respectivement.
